# EUROPEAN PATENT SPECIFICATION

(11) **EP 2 205 182 B1**
(45) Date of publication and mention of the grant of the patent: **23.12.2015**
(21) Application number: 08838016.7
(22) Date of filing: 10.10.2008
(51) Int. Cl.: A61F 2/04, A61B 5/03, A61B 5/00, A61B 17/11

(54) **ARTIFICIAL INTESTINE SECTION**
KÜNSTLICHER DARMABSCHNITT
SECTION D'INTESTIN ARTIFICIEL

(30) Priority: 11.10.2007 US 960715 P; 11.10.2007 US 960716 P; 12.10.2007 US 960765 P
(43) Date of publication of application: 14.07.2010
(73) Proprietor: Kirk Promotion LTD., 114 79 Stockholm (SE)
(72) Inventor: FORSELL, Peter, CH-6300 Zug (CH)
(74) Representative: Klunker . Schmitt-Nilson . Hirsch
(86) International application number: PCT/EP2008/008589
(87) International publication number: WO 2009/046997

(56) References cited:
- WO-A-2004/087233
- WO-A-2006/014496
- WO-A1-02/39959
- FR-A- 2 248 015
- US-A- 5 261 898
- US-A1- 2004 098 113
- US-A1- 2004 122 527
- US-A1- 2004 220 516
- US-B1- 6 402 767

## Description

### Background of the invention

The present invention relates to a system and method for treating a patient having a disorder related to the patient's intestine. Such disorder may be caused by injury, birth defect, cancer or other diseases, such as constipation or incontinence.

In an attempt to overcome such disorders, many different solutions have been proposed. These solutions often include surgery, in particular where a portion of the intestine has to be removed. The reason for such operation may be colorectal cancer, perforated diverticulitis or other kinds of diseases, such as ulceros colitis or Crohns disease. For instance, in the case of ileostomy, jejunostomy, colostomy and rectostomy operations the small intestine (jejunum or ileum) or the large intestine (colon or rectum) is cut and the open end of the healthy portion of the intestine is reattached either to a surgically created stoma in the patient's abdominal wall or, where possible, to the patient's rectum or anus or to tissue adjacent the patient's anus.

The problem then arises to control the intestinal contents flow and, more particularly, to prevent feces from exiting the patient's body uncontrolled. The patient is typically required to excrete into a colostomy bag. This is obviously inconvenient and, in addition, may cause skin irritation since such a bag arrangement requires an adhesive plate to be attached to the patient's skin in order to render the bag liquid tight.

US patent no. 4,222,377 suggests the use of an inflatable artificial sphincter comprising a cuff around the anal or urethral canal. A manually operated pump is implanted in the patient's scrotum for inflating and deflating the artificial sphincter.

Similarly, US patent no. 5,593,443 discloses an artificial hydraulic anal sphincter under voluntary control. More specifically, the patient may actuate a mechanical or electrical pump for inflating and deflating a cuff. The cuff consists of two parts positioned on opposite sides of the intestine and pressing the intestinal walls together when inflated.

US 6,752,754 B1 discloses an artificial rectum for replacing a portion of a patient's rectum. An inlet of the artificial rectum is operatively connected to the distal, cross-sectional end of the patient's large intestine and communicates fecal matter to a macerator-type pump that discharges the feces through an outlet of the artificial rectum connected to the patient's anus. The pump includes a helical screw-type impeller, which when rotated creates shearing effects on the feces, causing it to move down the thread of the screw impeller and discharge through the patient's anus.

Similarly, WO 02/39959 A1 discloses a receptacle for collecting intestinal contents, the receptacle being designed for surgical implant in the abdominal cavity. The receptacle comprises a connection device for connecting to the anastomosed end of the intestine and an emptying device secured to the abdominal wall of the patient to close the passage through the abdominal wall.

A more sophisticated device for replacing all or part of a colon or large bowel is described in US 2004/0122527 A1. Here the collecting device comprises a flexible tube in which fecal matter is collected and an implantable pump acting from outside onto the tube so as to advance the fecal matter through the tube by sequentially compressing sections of the tube similar to the peristaltic movement of a natural bowel.

### Summary of the invention

It is an object of the present invention to provide an improved system for treating a patient having a disorder related to the patient's intestine.

### LATERALLY CONNECTED ARTIFICIAL INTESTINE SECTION

The present invention provides an artificial intestine section adapted to be implanted inside the patient's body. The artificial intestine section of the present invention has a first open end portion and a second open end portion in flow communication with one another, wherein an artificial reservoir is provided between the first and second end portions for receiving and temporarily collecting therein intestinal contents supplied through the first open end portion. At least the first open end portion is adapted to being connected to a surgically created lateral opening in a wall of the patient's intestine. Thus, rather than connecting the reservoir to the cross-sectional end of the patient's intestine, it will be connected to a lateral opening in the patient's intestinal wall, and for this purpose the respective end portion of the artificial intestine section is specifically adapted.

By connecting the artificial intestine section laterally to the intestine, forces caused by the peristaltic movement of the intestine and acting on the artificial intestine section of the intestine are largely avoided. More specifically, where the artificial intestine section is connected to the cross-sectional opening of the intestine, the peristaltic waves of the intestine tend to pull the intestine off of the connection between the intestine and the artificial intestine section. As compared to this, where the artificial intestine section is attached to an opening in the lateral wall of the intestine, the peristaltic waves pass the artificial intestine section substantially without any impact on the connection between the intestinal wall and the artificial intestine section.

The second open end portion of the artificial intestine section may be adapted to being connected to a surgically created stomy or to the patient's rectum or anus or to tissue adjacent the patient's anus. Alternatively, since direct contact of the artificial intestine section with the patient's skin may cause inflammation and might not be acceptable on the long run for many reasons, it is likewise possible to adapt the second open end portion of the artificial intestine section so as to being connected to a portion of the large intestine or to a portion of the small intestine, as the case may be, and to connect that portion to the patient's rectum or anus or to tissue adjacent the patient's anus or to use that portion for creating a stomy.

### BY-PASS ARRANGEMENT

Alternatively, both the first and second open end portions can be adapted to being connected to a surgically created lateral opening in a wall of the patient's intestine.

### STRUCTURE OF ATTACHMENT

In order to securely attach the artificial intestine section to the lateral opening, at least the first open end portion may comprise a shoulder portion formed around the end portion for lateral connection to the patient's intestinal wall. Preferably, at least a part of the shoulder portion extends laterally from the artificial intestine section by 3 mm to 20 mm. Furthermore, the shoulder portion preferably has a curved cross section, so as to generally conform to the intestinal wall when laterally attached thereto. An open end portion adapted in this way can advantageously be attached to the intestinal wall from the outside thereof.

According to an improved embodiment, the shoulder portion may be split into an upper and a lower shoulder portion with a gap between the upper and lower shoulder portions for accommodating intestinal wall tissue therein. The lower shoulder portion, if suitably adapted, can then be placed inside the patient's intestine through the surgically created lateral wall opening, whereas the upper shoulder portion will be placed outside the intestinal wall.

In order to allow the lateral wall opening to be easily stretched over the lower shoulder portion when the lower shoulder portion is advanced there through and yet in order to have a large contact area between the intestinal wall and the shoulder portion, the upper shoulder portion may be made larger than the lower shoulder portion. Thus, the surface area of the upper shoulder portion contacting the intestinal wall is also larger than the surface area of the lower shoulder portion contacting the intestinal wall.

The open end portion for lateral connection to the patient's intestinal wall may be adapted to being connected to the patient's intestinal wall by gluing. For instance, it may have a particular rough surface structure for the glue to better adhere.. Also, the open end portion may be adapted to being connected to the patient's intestinal wall by sewing. For instance, a certain area of the shoulder portion may be perforated for stitching through the perforations or may be made from a material which is easy to penetrate with a needles. Similarly, the open end portion may specifically adapted to being connected to the patient's intestinal wall by stapling.

Preferably, at least the first open end portion is made from a biocompatible material. The biocompatible material of the open end portion may comprise at least one material of the following group of materials: titanium, stainless steel, ceramics, biocompatible polymer material. More specifically, the biocompatible polymer material may comprise at least one polymer of the following group of polymers: polytetrafluoroethylene, silicone, polyurethane, expanded polytetrafluoroethylene (ePTFE).

Also, at least the first open end portion preferably comprises a multilayer material. For instance, it is advantageous when the open end portion comprises a porous ingrowth layer that allows ingrowth of living tissue. The ingrowth layer may have a net-like structure and is most preferably made from Dacron®.

### INTESTINAL CONTENT INTERACTING DEVICE WITHIN ARTIFICIAL INTESTINE SECTION

In a preferred embodiment, the artificial intestine section is adapted to directly or indirectly interact with intestinal contents contained in the artificial intestine section between the first and second open end portions thereof.

### RESERVOIR

In its simplest form, the at least one element of the artificial intestine section comprises said artificial reservoir between the first and second open end portions for receiving and temporarily collecting therein intestinal contents supplied through the first open end portion.

### FLOW CONTROL DEVICE

In a more advanced embodiment, the at least one element may comprise, in addition to the reservoir, a flow control device adapted to control flow of intestinal contents from the artificial intestine section through the second open end portion. The flow control device is preferably adapted to prevent flow of intestinal contents from the artificial intestine section through the second open end portion.

### EXIT VALVE AS FLOW CONTROL DEVICE

The flow control device preferably comprises at least one valve, including an exit valve preventing intestinal contents flow through the second open end portion in its closed position. Preferably, the exit valve is a normally closed valve so that no energy is needed to keep the valve closed during the system's inactive periods.

### ENTRY VALVE AS AN ADDITITONAL PART OF THE FLOW CONTROL DEVICE

In addition, the flow control device may comprise an entry valve allowing intestinal contents to flow towards the reservoir in its open position. This can be advantageous particularly during the emptying of the reservoir, when the entry valve should be closed. Therefore, the entry valve is preferably a normally open valve. Accordingly, the exit valve and the entry valve are preferably adapted to cooperate such that when one of the two valves is closed, the respective other valve is open, and vice versa.

### VALVE TYPES

As regards the various valve types that may be employed, the at least one valve may e.g. comprise a central opening which is normally closed by resilient means that can be urged apart mechanically by inserting a conduit through the central opening so as to open the central opening of the valve. In the simplest embodiment, the valve may be opened by mechanical force, such as by inserting a tube from outside the patient's body through the valve. The valve in this case can be a simple non-return valve.

According to a more complex embodiment, the at least one valve may comprise a compartment with a variable volume adapted to open and close the valve by changing the compartment's volume. Advantageously, the at least one valve comprises at least one passage for filling and emptying the compartment with hydraulic fluid. The compartment preferably has at least one flexible wall defining an opening for the intestine or a conduit of the reservoir to pass through, the opening being adapted to close upon increase of the compartment's volume.

According to a different embodiment, the at least one valve may be a flap valve permanently implanted inside the patient's intestine. The flap valve may for instance comprise a rotatable disc.

### EXTRA VALVE SEPARATE FROM ARTIFICIAL INTESTINE PIECE

While the valve or valves preferably make an integral part of the artificial intestine section, the artificial intestine section may further comprise one or more extra valves adapted to control flow of intestinal contents in a natural section of the patient's intestine upstream and/or downstream the artificial intestine section. The extra valve may be rigidly connected to the artificial section but may as well form a completely separate part, in which case the artificial intestine section and the extra valve together rather form a "system". The extra valve is adapted to being implanted inside the patient's body outside a section of the patient's natural intestine and comprises at least one element adapted to act on the natural intestine section from the outside thereof so as to prevent intestinal contents flow through the natural intestine section. This valve arrangement does not require any surgery on the respective part of the natural intestine when the valve is implanted.

The extra valve may comprise at least one electrical stimulation device adapted to electrically stimulate muscle or neural tissue of an intestine section so as to cause at least partial contraction of the natural intestine section. This is a very gender way of constricting the intestine. The stimulation device preferably comprises at least one electrode adapted to apply electric pulses to the natural intestine section.

It is particularly advantageous to make use of a stimulation device which is adapted to stimulate different portions of the intestine section over time. Thus, different portions of the intestine section can be constricted by stimulation at different times in any predetermined stimulation pattern, thereby giving the intestine portions currently not stimulated time to recover and, thus, improving the blood circulation in the respective intestine section.

Furthermore, the stimulation device can specifically be adapted to stimulate, over time, the different portions of the intestine section in a wave like manner in a direction opposite to natural intestinal contents flow. As a result, the valve counteracts the natural intestinal contents flow, thereby improving the valve's closing function.

Alternatively, or preferably in addition to the stimulation device, the at least one valve may comprise a constriction device implanted in the patient's body for at least partly constricting the natural intestine section mechanically from outside the natural intestine section. Where the stimulation device is combined with the constriction device, the stimulation device and the constriction device preferably act on the same intestine section. In that case, it is advantageous if the constriction device in its normal condition constricts the natural intestine section only partly, in order not to damage the intestine over time. Complete constriction and, thus, closing of the intestine may then be obtained by additionally stimulating the natural intestine section in a manner as described before.

In addition, when constriction of the intestine section caused by the constriction device is released, the stimulation device may, if accordingly adapted, be used to pump intestinal contents along the natural intestine section by, over time, stimulating the different portions of the natural intestine section in a wave like manner in a direction of natural intestinal contents flow. In this situation, the valve may incorporate the additional function of a pump for actively supporting the discharge of feces from the human body.

### PUMP AS PART OF THE IMPLANTABLE FLOW CONTROL DEVICE

The flow control device may comprise a pump for advancing intestinal contents through the second open end portion to outside the artificial intestine section. Where the artificial intestine section comprises a reservoir, the pump may be adapted for emptying the reservoir. A variety of different structures may be realized.

For instance, the reservoir may be formed by a bellow, said bellow having an end wall closing the bellow at one end thereof. The end wall may then make part of the pump such that a volume of the bellow is reduced upon advancement of said end wall. Preferably, bellow is made of a resilient material so as to urge the bellow into a normally expanded position.

In another embodiment, the pump may comprise a movable piston, with a front end of the piston extending into the reservoir such that a volume of the reservoir is reduced upon advancement of the piston. Preferably, the piston is spring loaded so as to urge the piston into a normally retracted position.

Alternatively, the pump may be adapted for being permanently arranged inside the reservoir.

In a further alternative, the reservoir may have a flexible wall and the pump is adapted for emptying the reservoir by squeezing the reservoir. In this case, the pump may e.g. include a constriction device adapted to alternately constrict and release sections of the reservoir so as to pump intestinal contents along the reservoir by, over time, constricting different sections of the reservoir in a wave like manner. More specifically, the reservoir may have a tube-like form and a roller pump may be used as the pump acting on the tube-like reservoir from the outside thereof.

### MOTOR

Where the valves or pump or any other element of the flow control device is not or not only manually drivable, at least one motor can be provided for automatically driving at least one energy consuming part of the flow control device. The motor is preferably arranged to be driven by electric or electromagnetic energy.

A motor in the sense of the present invention is a device that transforms energy other than mechanical energy into mechanical energy. While a pump in the sense of the present invention is a device for advancing liquid or pasty material, a pump may at the same time be a motor in certain circumstances, such as where the transformation of energy into mechanical energy causes advancement of the liquid or pasty material without any intervening mechanical means such as a piston, bellow or the like.

For instance, the at least one motor can be arranged for driving at least one of the valve or valves, respectively, between its closed and open position. Also, the at least one motor can be arranged for driving the pump.

A manually operable switch may be provided for activating the at least one motor, the switch being preferably arranged for subcutaneous implantation so as to be operable from outside the patient's body.

### ENERGY SOURCE

The artificial intestine section may be combined with further components to form a system. The further components may be integrated in the artificial intestine section to be implanted along therewith or may be separate from the artificial intestine section to be implanted separately or not to be implanted at all.

The system may for instance comprise an energy source for supplying energy directly or indirectly to at least one energy consuming part of the system. Preferably, the energy source includes a battery or an accumulator, such as one or more of a rechargeable battery and a capacitor, as an energy storage means. The energy storage means is advantageously adapted for being implanted inside the patient's body, more preferably as a part of the artificial intestine section.

### WIRELESS ENERGY TRANSMISSION

Energy is preferably transmitted wirelessly. Thus, where the energy source is provided for supplying energy directly or indirectly to at least one energy consuming part of the system, the energy source may comprise a wireless energy transmitter adapted to wirelessly transmit energy from outside the patient's body to the at least one energy consuming part. Alternatively, where the energy source includes a battery or an accumulator, in particular one which is implanted in the patient's body, the energy source may comprise a wireless energy transmitter adapted to wirelessly transmit energy from outside the patient's body to the energy storage means.

### ENERGY TRANSMISSION FEEDBACK

A feedback subsystem, which can make part of a control device described subsequently, can advantageously be provided to wirelessly send feedback information related to the energy to be stored in the accumulator from inside the human body to the outside thereof. The feedback information is then used for adjusting the amount of wireless energy transmitted by the energy transmitter. Such feedback information may relate to an energy balance which is defined as the balance between an amount of wireless energy received inside the human body and an amount of energy consumed by the at least one energy consuming part. Alternatively, the feedback information may relate to an energy balance which is defined as the balance between a rate of wireless energy received inside the human body and a rate of energy consumed by the at least one energy consuming part.

Also, the transmission of energy from the energy storage means to the at least one energy consuming part may be performed wirelessly by means of an accordingly adapted wireless energy transmitter.

Preferably, in order to reduce the number of parts and possibly increase the system's efficiency, the energy consuming part can be adapted to directly transform the wirelessly transmitted energy into kinetic energy. Otherwise, it will be necessary to provide an implantable energy transforming device for transforming the wireless energy, preferably into electric energy. In this case, it is further preferred to set up the system such that the energy consuming part is driven with the electric energy, as said energy transforming device transforms the wireless energy into the electric energy.

The energy transmitter can be adapted to generate an electromagnetic field, a magnetic field or an electrical field. The wireless energy may be transmitted by the energy transmission device by at least one wireless signal. More specifically, the energy transmitter may be adapted to transmit the energy by at least one wireless energy signal, which may comprise an electromagnetic wave signal, including at least one of an infrared light signal, a visible light signal, an ultra violet light signal, a laser signal, a microwave signal, an X-ray radiation signal, and a gamma radiation signal. Also, the wireless energy signal may comprise a sound or ultrasound wave signal. Furthermore, the wireless energy signal may comprise a digital or analog signal or a combination thereof.

### GALVANIC ENERGY TRANSMISSION

Where energy is not transmitted wirelessly, galvanic coupling elements should be provided at least between the energy source and the motor for transmitting energy to the motor in contacting fashion.

### CONTROL UNIT

It is advantageous to provide a control unit adapted to directly or indirectly control one or more elements of the system, such as for controlling opening of the exit valve and/or closing of the entry valve, in particular in a manner such that when one of the two valves is closed, the respective other valve is open, and vice versa. The control unit can also be adapted to control actuation of the pump.

The control unit is preferably operable by the patient, e.g. particularly in order to empty the reservoir.

At least part of the control unit may be adapted to be implantable in the patient's body. For instance, a manually operable switch may be provided for activating the control unit, the switch preferably being arranged for subcutaneous implantation so as to be operable from outside the patient's body. Also, the control unit may comprise a first part adapted for implantation in the patient's body and a second part adapted to cooperate with the first part from outside the patient's body. In this case, the control unit can be adapted to transmit data from the external second part of the control unit to the implanted first part of the control unit in the same manner as energy is transmitted to the at least one energy consuming part.

That is, the second part of the control unit may be adapted to wirelessly transmit a control signal to the implantable first part of the control unit for controlling the at least one energy consuming part from outside the patient's body. Also, the implantable first part of the control unit may be programmable via the second part of the control unit. Furthermore, the implantable first part of the control unit may be adapted to transmit a feedback signal to the second part of the control unit.

### SENSOR

Furthermore, a physical parameter sensor adapted to directly or indirectly sense a physical parameter of the patient can be provided. The physical parameter sensor may be adapted to sense at least one of the following physical parameters of the patient: a pressure within the artificial intestine section, a pressure within the patient's intestine, an expansion of the artificial intestine section, a distension of an intestinal wall of the patient's intestine, a movement of the intestinal wall.

Similarly, a functional parameter sensor adapted to directly or indirectly sense a functional parameter of the system can be provided, wherein the functional parameter sensor may be adapted to sense at least one of the following functional parameters of the system: a pressure against a part of the system such as the artificial intestine section, a distension of a part of the system such as a wall of the artificial intestine section, an electrical parameter such as voltage, current or energy balance, a position or movement of a movable part of the system.

Preferably, an indicator is coupled to the sensor or sensors, the indicator being adapted to provide a signal when a sensor senses a value for the parameter beyond a predetermined threshold value. The sensor signal may comprise at least one of the following types of signals: a sound signal, a visual signal.

### INTESTINAL CONTENTS COLLECTING DEVICE (WITH "EXTERNAL" PUMP)

Where the artificial intestine piece comprises a reservoir, in a simple way, an intestinal contents collecting device may used to be temporarily applied from outside the patient's body when the reservoir is to be emptied. According to a preferred embodiment, the collecting device may comprise a front open end adapted to be applied towards the exit valve so as to provide a flow passage from the exit valve towards the collecting device. More specifically, the collecting device front open end is preferably adapted to be applied to the exit valve so as to open the valve and thereby provide said flow passage towards the collecting device. Where the exit valve is normally closed by resilient means, said front open end is adapted to be inserted through the central opening of the exit valve so as to urge apart the resilient means normally closing the central opening.

The collecting device preferably comprises a suction pump, which may comprise a piston-cylinder-arrangement. The suction pump may be adapted to be driven manually, in particular where it is intended for use as a back-up pump for a situation where the pump of the flow control device is out of operation. However, preferably a motor is connected to the suction pump for driving the pump automatically.

### METHOD OF TREATMENT (IMPLANTATION)

The invention does not only relate to the artificial intestine section and systems described above, but also to a method of treating a patient having a disorder related to the patient's intestine.

### LATERAL CONNECTION

Connecting at least the first open end portion of the artificial intestine section laterally to the patient's intestine involves the following steps of a surgical method of treating a patient:
- cutting the patient's skin and abdominal wall,
- dissecting an area of the patient's intestine,
- surgically creating at least one opening in a wall of the dissected intestinal area so as to create an artificial lateral intestinal opening,
- providing an artificial intestine section having a first open end portion and a second open end portion in flow communication with one another and affixing the first open end portion to the lateral intestinal opening so as to be in flow communication therewith, and
- suturing the abdominal wall and skin.

A corresponding laparoscopic surgical method of treating a patient would comprise the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferably through a second trocar,
- dissecting an area of the intestine,
- surgically creating at least one opening in a wall of the dissected intestinal area so as to create an artificial lateral intestinal opening,
- providing an artificial intestine section having a first open end portion and a second open end portion in flow communication with one another and affixing the first open end portion to the lateral intestinal opening so as to be in flow communication therewith,
- extracting the instruments, camera and trocar, and in relation thereto
- suturing, if necessary, the abdominal wall and permanently closing the skin.

### CLOSING THE INTESTINE BY SEWING

The dissected portion may be permanently closed at a location downstream of the lateral intestinal opening so as to create an upstream part of the intestine including the lateral intestinal opening and a downstream part of the intestine, and the second open end portion of the artificial intestine section may be affixed to the downstream intestinal part, preferably again to a lateral opening in the wall of the downstream intestinal part. The downstream intestinal part may be connected to a surgically created stomy or to the patient's rectum or anus or to tissue adjacent the patient's anus. The step of permanently closing the patient's intestine preferably comprises sewing and/or stapling the intestinal wall so as to form a dead end.

### DIVIDING THE INTESTINE

The patient's intestine may alternatively be divided and the artificial intestine piece may be placed between the resulting upstream and downstream intestinal parts. This would require the following steps:
- dissecting a portion of the dissected intestinal area downstream of the lateral intestinal opening such that intestinal mesentery connected to the dissected portion is opened in such a way that supply of blood through the mesentery to the dissected intestinal area is maintained as far as possible on both sides of the dissected portion,
- dividing the patient's intestine in the dissected portion so as to create an upstream part of the intestine with the lateral intestinal opening and a downstream part of the intestine, said downstream intestine part being separate from the upstream intestine part and having a cross-sectional opening at the upstream side thereof, wherein the mesentery maintains a tissue connection between the upstream and downstream intestine parts,
- affixing the second open end portion of the artificial intestine section to the downstream intestine part, and
- permanently closing the upstream intestine part at a location downstream of the lateral intestinal opening.

### LATERAL FRONT AND LATERAL END CONNECTION (BY-PASS)

As mentioned before, preferably not only the first open end but also the second open end of the artificial intestine section are connected to a lateral opening in the patient's intestinal walls. This would involve the following steps to be performed on the downstream intestinal part:
- surgically creating an opening in a wall of the downstream intestinal part at an upstream end thereof so as to create a second artificial lateral intestinal opening, and
- affixing the second open end portion of the artificial intestine section to the second lateral intestinal opening so as to be in flow communication therewith.

The step of permanently closing the patient's intestine may then comprise sewing the intestinal wall with two rows of sutures or staples and cutting and dividing the intestine between the sutures or staples so as to form two dead ends.

Alternatively, where the method of implanting the artificial intestine section involves dividing the patient's intestine, the step of affixing the second open end portion of the artificial intestine section to the downstream intestinal part may comprise the steps of:
- surgically creating an opening in a wall of the downstream intestinal part at an upstream end thereof so as to create a second artificial lateral intestinal opening,
- affixing the second open end portion of the artificial intestine section to the second lateral intestinal opening so as to be in flow communication therewith, and
- permanently closing the cross-sectional opening at the upstream side of the downstream intestine part at a location upstream of the second lateral intestinal opening, e.g. by sewing and/or stapling.

### LATERAL FRONT CONNECTION AND STRAIGHT END CONNECTION

However, the second open end portion of the artificial intestine section can likewise be affixed to a cross-sectional upstream opening of the downstream intestinal part of the divided intestine so as to be in flow communication therewith.

### SLEEVE/BULGE CONNECTOR

The step of affixing the second open end portion of the artificial intestine section to a cross-sectional upstream opening of the downstream intestine part preferably comprises:
- inserting the second open end portion of the artificial intestine section into the upstream opening of the downstream intestine part, and
- placing a flexible sleeve so as to extend over both the downstream intestine part and second open end portion of the artificial intestine section such that the downstream intestine part is located intermediate the sleeve and the outer surface of the artificial intestine section.

Where the flexible sleeve is mounted on the outer surface of the second open end portion of the artificial intestine piece so as to be foldable upon itself, the step of placing the flexible sleeve so as to extend over both the downstream intestine part and second open end portion of the artificial intestine section comprises folding the flexible sleeve upon itself such that the downstream intestine part is located intermediate the folded sleeve.

Alternatively, or in addition, the step of affixing the second open end portion of the artificial intestine section to the cross-sectional upstream opening of the downstream intestine part may comprise:
- inserting the artificial intestine section having a bulge formed on the outside thereof into the upstream opening of the downstream intestine part so that the downstream intestine part extends over the bulge from one side of the bulge, and
- advancing a blocking ring over the downstream intestine part towards the bulge from the respective other side of the bulge such that the downstream intestine part is located intermediate the outer surface of the artificial intestine section and the blocking ring.

The afore-mentioned second open end portion of the artificial intestine section with a sleeve or with a bulge serve to improve the strength of the connection against axial forces which may e.g. result from the peristaltic movement of the intestine and tend to pull on the intestine. The second open end portion of the artificial intestine section may also combine a sleeve and a bulge.

### EXIT THROUGH STOMA

As mentioned before, the downstream intestinal part may be connected to a surgically created stomy or to the patient's rectum or anus or to tissue adjacent the patient's anus. In the case of a connection to a stomy, this would involve the following steps:
- cutting the patient's skin and abdominal wall so as to create an opening for an intestinal stomy,
- dissecting the area of the stomy opening,
- dividing the intestine at a location downstream of the artificial intestine piece so as to create an upstream natural intestine section having a cross-sectional opening at the downstream end thereof and a downstream natural intestine section,
- dissecting the mesentery of the upstream natural intestine section in the area of the cross-sectional opening thereof to prepare for creating the intestinal stomy,
- advancing the downstream end of the upstream natural intestine section through the abdominal wall and skin, and
- suturing the cross-sectional opening of the upstream natural intestine section to the skin with the intestinal mucosa turned inside out, thereby achieving the intestinal stomy.

### EXIT THROUGH ANUS

In the case of a connection to the patient's anus or to tissue adjacent the patient's anus, this would involve the following steps:
- dividing the intestine at a location downstream of the artificial intestine piece so as to create an upstream natural intestine section having a cross-sectional opening at the downstream end thereof and a downstream natural intestine section leading to the patient's anus,
- dissecting the area of the patient's anus and surgically separating the downstream natural intestine section from the patient's anus, whereas the steps of dividing the intestine and separating the intestine section leading to the patient's anus can alternatively be carried out in reversed order,
- dissecting the mesentery of the upstream natural intestine section in the area of the cross-sectional opening at the downstream end thereof to prepare for connecting the upstream natural intestine section to the patient's anus or tissue adjacent the patient's anus,
- advancing the downstream end of the upstream natural intestine section through the patient's anus, and
- suturing the cross-sectional opening of the upstream natural intestine section to the patient's anus or tissue adjacent the patient's anus.

Depending upon the circumstances, the step of dividing the intestine so as to form the upstream natural intestine section may be performed either on the patient's small intestine or on the patient's large intestine.

### STRUCTURE OF ATTACHMENT

Where the first open end portion of the artificial intestine section and possibly also the second open end portion are to be affixed to the lateral intestinal opening so as to be in flow communication therewith, this may comprise the step of connecting the afore-mentioned shoulder portion, which is formed around the open end portion, to the patient's intestinal wall so as to surround the lateral intestinal opening. In particular, the step of affixing the open end portion to the intestine may comprise attaching the shoulder portion to the patient's outer intestinal wall.

Alternatively, where the shoulder portion is split into an upper and a lower shoulder portion with a gap between the upper and lower shoulder portions, the step of affixing the open end portion of the artificial intestine section to the lateral intestinal opening may comprise placing the lower shoulder portion inside the patient's intestine and the upper shoulder portion outside the patient's intestine such that the intestinal wall tissue is accommodated in the gap.

The step of affixing the open end portion of the artificial intestine section to the lateral intestinal opening may comprise gluing, sewing and/or stapling the open end portion to the patient's intestinal wall.

As a material for the first open end portion at least one biocompatible material from the following group of materials may be selected: titanium, stainless steel, ceramics, biocompatible polymer material, wherein the biocompatible polymer is preferably selected from the following group of polymers: polytetrafluoroethylene, silicone, polyurethane, expanded polytetrafluoroethylene (ePTFE).

More specifically, a multilayer material may be selected for the first open end, in particular one having a porous ingrowth layer allowing ingrowth of living tissue. The ingrowth layer is preferably chosen to have a net-like structure.

### INTESTINAL CONTENT INTERACTING DEVICE WITHIN ARTIFICIAL INTESTINE PIECE / RESERVOIR / FLOW CONTROL DEVICE

As mentioned before, the artificial intestine section or system may comprise at least one element adapted to directly or indirectly interact with intestinal contents contained in the artificial intestine section between the first and second open end portions thereof. This element will be implanted along with the artificial intestine section. As also mentioned before, the element may comprise a reservoir for receiving and temporarily collecting therein intestinal contents supplied through the first open end portion and/or a flow control device adapted to control flow of intestinal contents from the artificial intestine section through the second open end portion.

Again, the flow control device may comprise an exit valve preventing intestinal contents flow through the second open end portion in its closed position and may additionally comprise an entry valve allowing intestinal contents to flow through the first open end portion into the artificial intestine section in its open position.

Alternatively or in addition, as also mentioned before, the flow control device may comprise a pump for advancing intestinal contents through the second open end portion to outside the artificial intestine section. Where the pump comprises a manually operable switch for activating the pump, the method of implantation may further comprise the step of implanting the switch subcutaneously so as to be operable from outside the patient's body.

### MOTOR

Again, at least one motor may be implanted in the patient's body either separately or, more preferably, integrally with the artificial intestine section and may be arranged for automatically driving one or more energy consuming part of the flow control device. Where the motor comprises a manually operable switch for activating the motor, the method of implantation may further comprise the step of implanting the switch subcutaneously so as to be operable from outside the patient's body.

### ENERGY SOURCE / ENERGY TRANSMISSION

The method of implantation may further comprise the step of implanting inside the patient's body an energy source, possibly comprising energy storage means such as a battery or an accumulator as described before, for supplying at least one of the energy consuming parts with energy.

Where energy is transmitted wirelessly, for instance from outside the patient's body to inside the patient's body either to an energy consuming part and/or to the accumulator or from the accumulator to the energy consuming part, it may further be necessary to implant an energy transforming device for transforming the wireless energy into electric energy. Alternatively or in addition, galvanic coupling elements may be implanted, e.g. for transmitting energy to the energy consuming part in contacting fashion from outside the patient's body and/or from the implanted energy source.

### CONTROL UNIT

Furthermore, as mentioned previously, at least a part of a control unit may be implanted inside the patient's body adapted to directly or indirectly control one or more of the elements that have also been implanted in the patient's body. Where the control unit comprises a manually operable switch for activating the control unit, the method of implantation may further comprise the step of implanting said switch subcutaneously so as to be operable from outside the patient's body.

### SENSOR

As mentioned before, one or more physical and/or functional parameter sensors may be implanted to directly or indirectly sense physical and/or functional parameters inside the patient and in the system implanted inside the patient. Where the sensor is a pressure sensor, it may be placed in the artificial intestine section or the patient's natural intestine so as to sense the pressure within the artificial intestine section or patient's natural intestine, respectively. Where the sensor is a tension sensor, it may be placed in contact with the artificial intestine section or the patient's intestine so as to sense an expansion of the artificial intestine section or patient's natural intestine, respectively. Where the sensor is a movement sensor, it may be placed in contact with the artificial intestine section or the patient's natural intestine so as to sense movement of the artificial intestine section or patient's natural intestine, respectively. The functional sensor may be adapted to measure at least one of the following functional parameters of the system: an electrical parameter such as voltage, current or energy balance or a stimulation parameter in relation to the system.

### USE

Once the artificial intestine section or system according to the invention has been properly installed, the flow control device can be used for emptying the reservoir implanted in the patient.

Accordingly, a method of treating a patient by means of the artificial intestine section which comprises at least one element adapted to directly or indirectly interact with intestinal contents contained in the artificial intestine section may comprise the step of actuating the at least one element so as to interact with the intestinal contents contained in the artificial intestine section between the first and second open end portions thereof.

### EXIT AND ENTRY VALVE

Where the at least one element comprises an exit valve preventing intestinal contents flow from the artificial intestine section through the second open end portion in its closed position, the method may further comprise the steps of opening the exit valve and then removing intestinal contents from the artificial intestine section. Furthermore, where the at least one element further comprises an entry valve allowing intestinal contents to flow through the first open end portion into the artificial intestine section in its open position, the method may further comprise the step of closing the entry valve before removing intestinal contents from the artificial intestine section.

In particular, the method of use may comprise the step of inserting a conduit from outside the patient's body into the artificial intestine section, thereby mechanically urging the exit valve to open.

### PUMP

Where the at least one element interacting with intestinal contents inside the artificial intestine piece comprises a pump, the method of use may further comprise the step of advancing intestinal contents from the artificial intestine section through the second open end portion thereof to outside the artificial intestine section by means of the pump. The pump may be activated by manually operating a subcutaneously arranged actuator from outside the patient's body.

Alternatively, a flow passage may be provided to extend from the artificial intestine section to an external collecting device and intestinal contents may then be removed from the artificial intestine section by means of a suction pump.

### MOTOR

The suction pump is preferably driven by means of a motor.

Furthermore, where the at least one element interacting with intestinal contents inside the artificial intestine piece comprises a motor, the method of use may further comprise the step of driving at least the exit valve between its closed and open positions and/or driving at least the pump by means of the motor. In either case, the motor is preferably activated by manually operating a subcutaneously arranged actuator from outside the patient's body.

### ENERGY

As mentioned before, energy may be transmitted from outside the patient's body to at least one implanted energy consuming part of the system, preferably in the form of wireless energy. This may involve the following additional steps:
- transforming the wirelessly transmitted energy into electric energy by means of an energy transforming device,
- storing the transformed energy in an energy storage means, and
- supplying the stored energy from the energy storage means to at least one implanted energy consuming part of the system.

Again, energy may be transmitted wirelessly from the storage means to the energy consuming part.

Preferably, at least part of the wirelessly transmitted energy is transformed into electric energy and used for the energy consuming part of the system, as said part of the wirelessly transmitted energy is transformed into the electric energy.

### CONTROL

Where a first part of a control unit for controlling at least one energy consuming part of the system is implanted inside the patient's body, the method of use may further comprise the step of using the external second part of the control unit to transmit data to the implanted first part of the control unit. Preferably, the data are transmitted to the implanted first part of the control unit in the same manner as energy is transmitted to the implanted energy consuming part. More particularly, the data are preferably transmitted wirelessly to the implanted first part of the control unit. This may involve a wireless control signal.

For instance, the implanted first part of the control unit can be programmed via the external second part of the control unit. Furthermore, a feedback signal may be transmitted from the implanted first part of the control unit to the external second part of the control unit.

### SENSOR

Where one or more of the afore-mentioned sensors are provided, the method of use may comprise the step of sensing a physical parameter in the patient's body and/or a functional parameter of the artificial intestine piece or system in the patient's body, such as one or more of the following parameters: a pressure within the artificial intestine section, a pressure within the patient's natural intestine, an expansion of the artificial intestine section, a distension of an intestinal wall of the patient's natural intestine, a movement of the patient's intestinal wall, a pressure against a part of the system such as the artificial intestine section, a distension of a part of the system such as a wall of the artificial intestine section, an electrical parameter such as voltage, current or energy balance, a position or movement of a movable part of the system.

A signal, such as a sound signal or a visual signal, may be provided when a value for the physical parameter sensed is beyond a predetermined threshold value.

The invention will now be described in more detail in context with some preferred embodiments of the invention as shown in the accompanying drawings.

### Brief description of the drawings

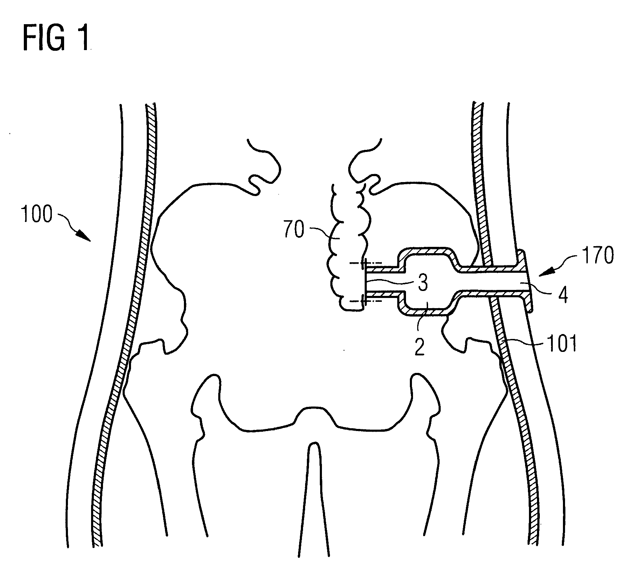

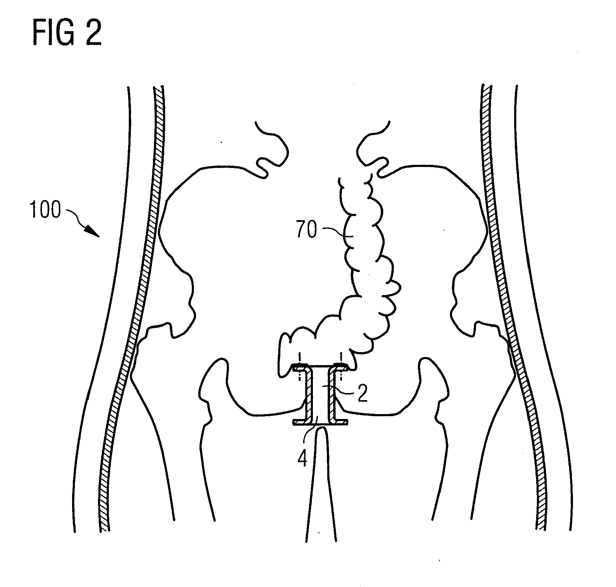

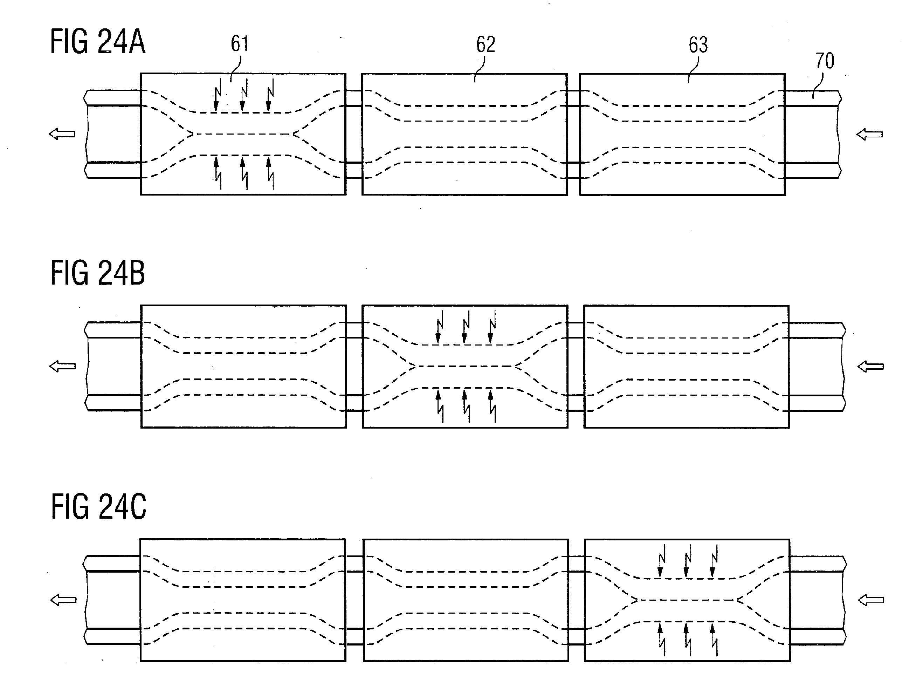

Figure 1 shows a system according to the present invention with an artificial intestine section 2 being implanted inside a patient's body 100 and having a first open end portion 3 connected to a surgically created lateral opening in a wall of the patient's intestine 70. The second open end portion 4 exits the patient's abdominal wall 101 forming a stomy 170. The artificial intestine section 2 is here shown as a black box and may include an artificial reservoir for intestinal contents, a motor, one or more valves, a pump and/or any other flow control device.
The system shown in Figure 2 corresponds to the one shown in Figure 1, however, with the second open end portion 4 of the artificial intestine section 2 exiting the patient's anus.

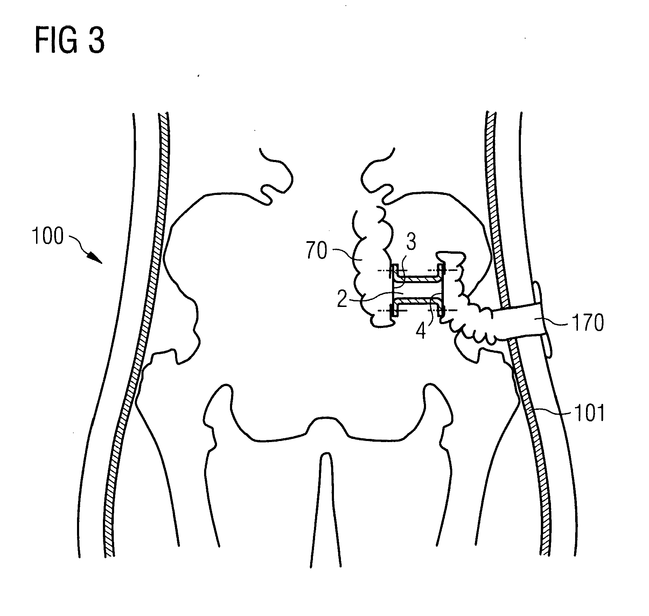

Figure 3 shows a system where both the first and second open end portions of the artificial intestine section 2 are attached to surgically created lateral openings in a wall of the patient's small 70 and/or large 80 intestine. The downstream part 80 of the intestine exits the patient's abdominal wall 101 forming a surgically created stomy 170. The downstream part 80 of the intestine may as well exit through the patient's anus.

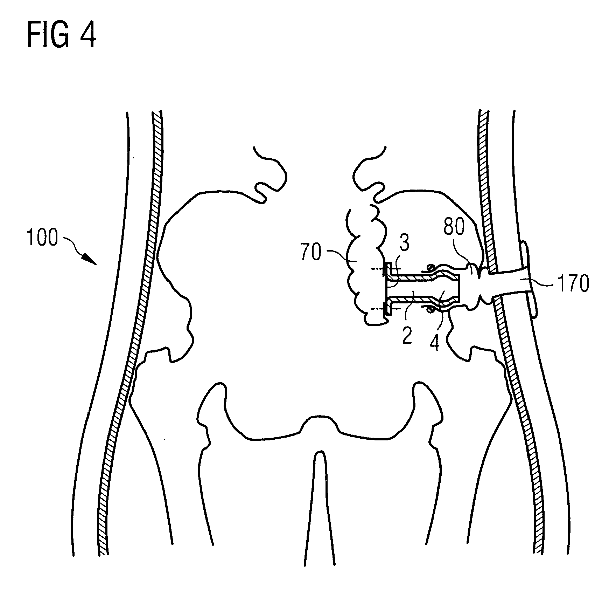

Figure 4 shows a similar system with the difference that the second open end portion 4 is connected to a cross-sectional opening of the patient's intestine, further leading to the surgically created stomy 170. The downstream part 80 of the intestine may as well exit through the patient's anus.

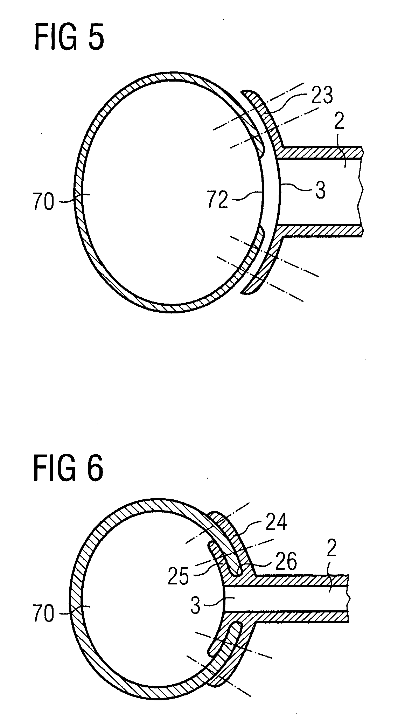

Figure 5 shows the structure of the first open end portion 3 of the artificial intestine section 2 for attaching the artificial intestine section 2 to the lateral opening 72 in the patient's intestine 70 by means of a shoulder portion 23 formed around the end portion 3. The end portion 3 is sewn to the intestine 70 and may additionally or alternatively be stapled and/or glued to the intestine 70.
Figure 6 shows an improved structure for lateral attachment to the intestine 70, wherein the shoulder portion is split into an upper 24 and a lower 25 shoulder portion forming a gap 26 to accommodate intestinal wall tissue therein. The surface area of the upper shoulder portion 24 is larger than the surface area of the lower shoulder portion 25.

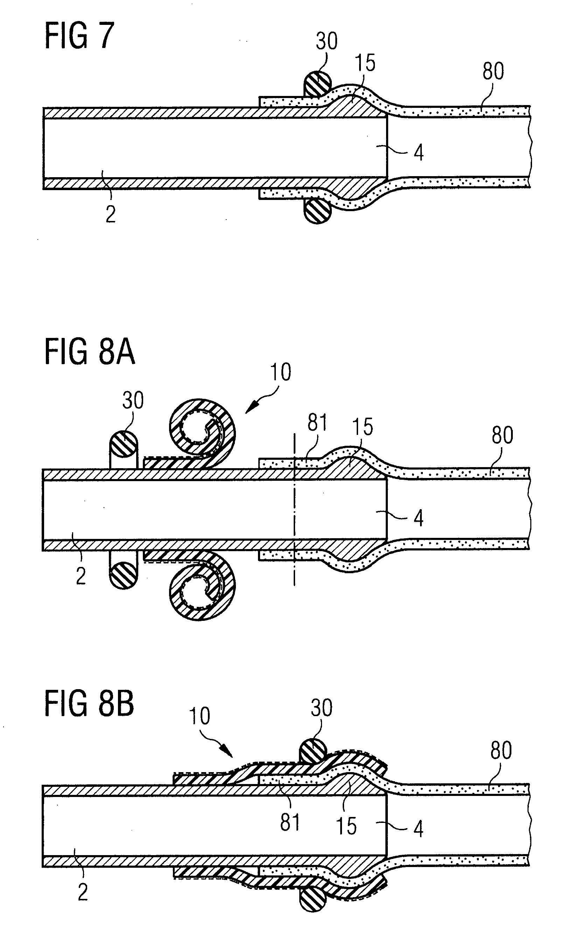

Figure 7 shows an enlarged view of a ring-and-bulge connection 15, 30 by which the artificial intestine section 2 and the patient's downstream intestinal part 80 are connected, as shown in Figure 4.
Figures 8A and 8B show the ring-and-bulge connection 15, 30 of Figure 7 in combination with a sleeve 10. The sleeve 10 is rolled upon itself and can be unrolled such that a part 81 of the intestine 80 is located intermediate the sleeve 10 and the conduit 2. Thereafter, the ring 30 is pushed over the sleeve 10 against the bulge 15.

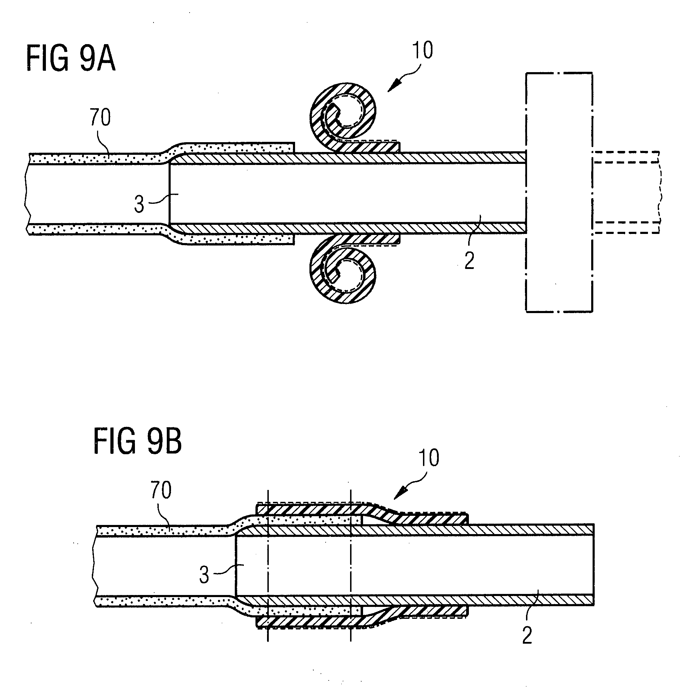

Figures 9A and 9B show a connection of the artificial intestine section 2 to the cross-sectional opening of the patient's intestine 70 similar to the connection shown in Figures 8A and 8B, however, without the bulge and the ring.

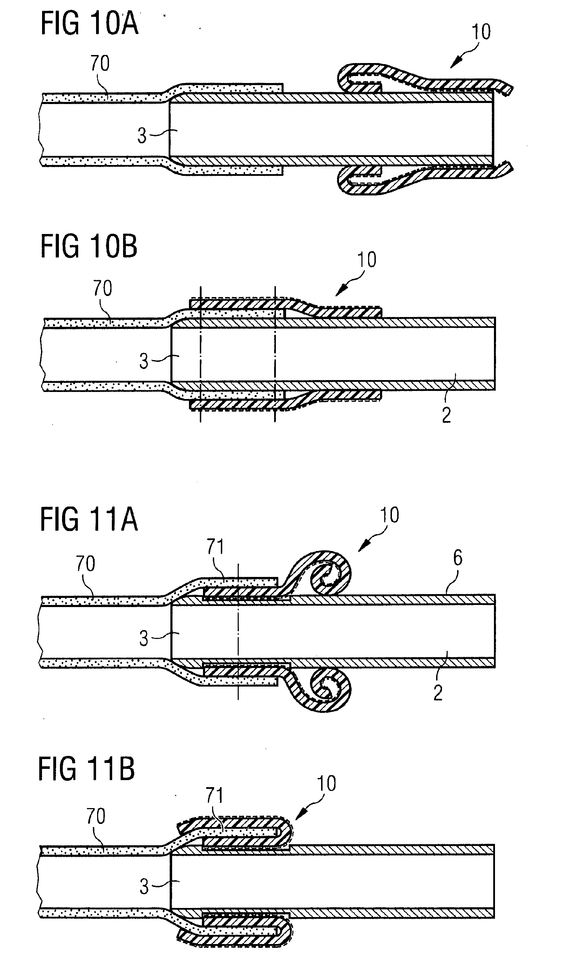

Figures 10A and 10B show an alternative to the connection in Figure 9A and 9B. Instead of unrolling the sleeve 10, it is simply pulled over the intestine 70.
Figures 11A and 11B show another sleeve connection. Here, the sleeve 10 is mounted on the outer surface 6 of the open end portion so as to be foldable upon itself. By folding the flexible sleeve 10 upon itself, a part 71 of the intestine 70 is located intermediate the folded sleeve 10.

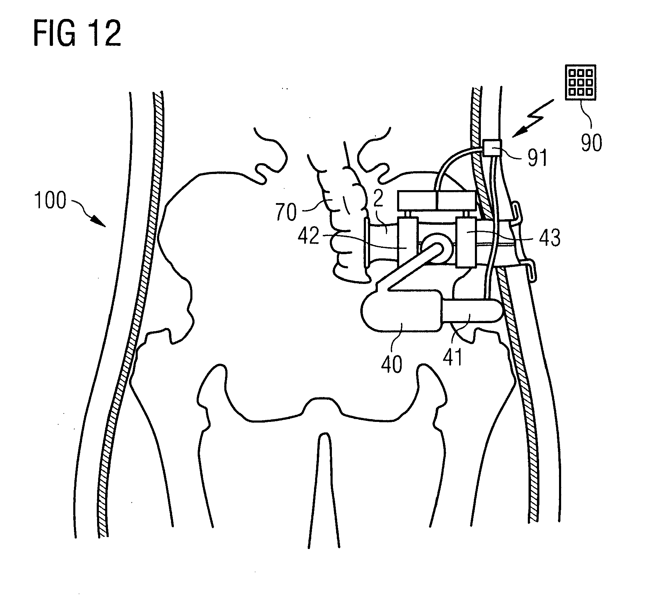

Figure 12 shows an embodiment of the artificial intestine section 2 with an artificial reservoir 40 and an entry valve 42 and exit valve 43 arranged upstream and downstream of the reservoir 40. The reservoir 40 is mounted with a pump 41 in a common housing and the pump 41 and the entry and exit valves 42, 43 are controlled by means of a control device, of which a part 91 is implanted inside the patient's body 100. Data are transmitted wirelessly between the external part 90 and implanted part 91 of the control unit. In addition, energy is wirelessly transmitted to the artificial intestine section 2 or to an accumulator also implanted in the patient's body 100 and galvanically connected here to the valves 42, 43 and pump 41.

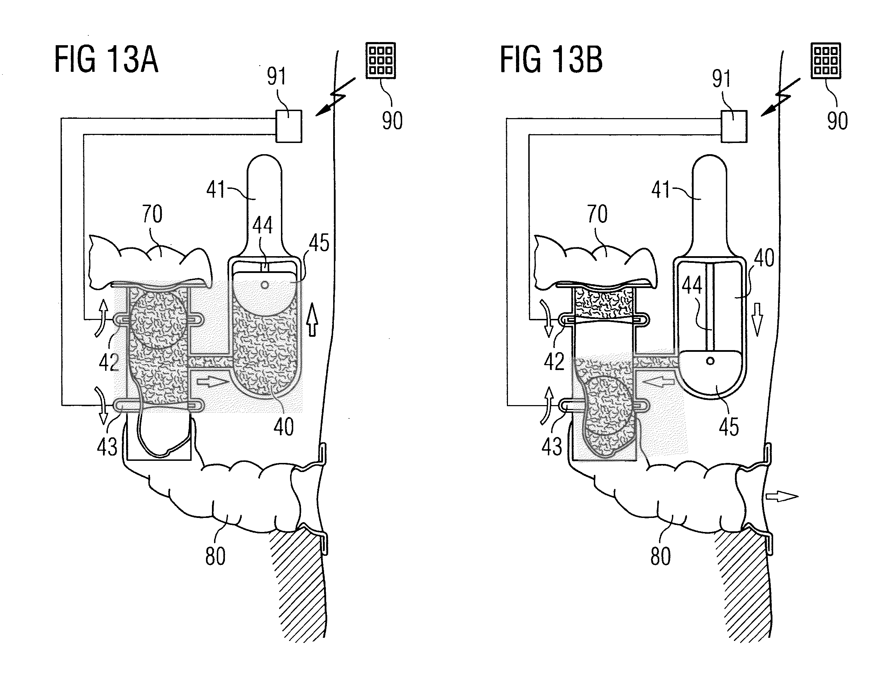

Figures 13A and 13B show a first embodiment of the structure of Figure 12 in more detail. The pump 41 comprises a moveable piston 44 with a front end 45 of the piston 44 extending into the reservoir 40 such that a volume of the reservoir 40 is reduced upon advancement of the piston 44. The piston 44 is spring loaded so as to urge the piston 44 into a normally retracted position. Furthermore, entry and exit valves 42, 43 are here realized as flap valves. The flap valves are controlled so that one valve is open while the other one is closed.

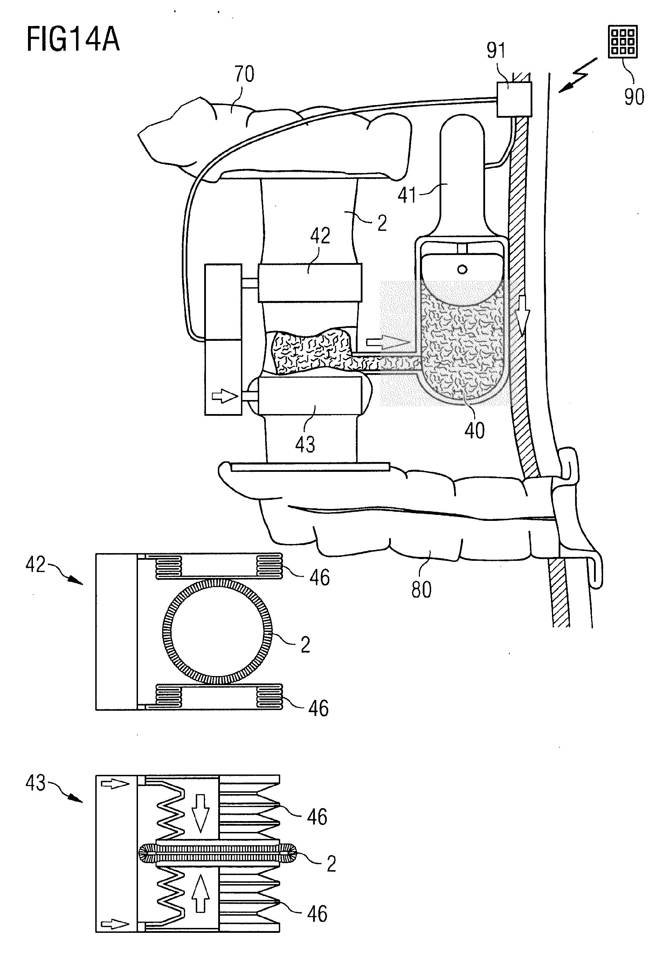

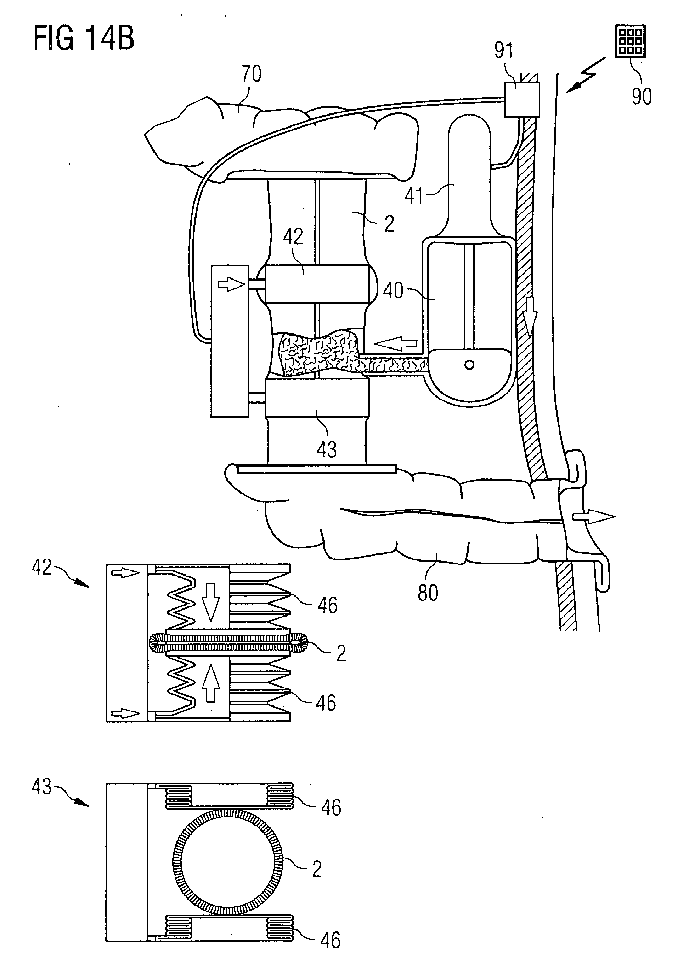

Figures 14A and 14B show a system similar to the one of Figures 13A and 13B. However, here the entry and exit valves 42, 43 comprise bellows 46 acting on the intestine from the outside so as to close the intestine by compression. In Figure 14A the bellows 46 of the exit valve 43 are expanded to compress the artificial intestine section 2 at the downstream side of the reservoir 40, whereas in Figure 14B the artificial intestine section 2 is closed by means of the bellows 46 of the entry valve 42 upstream of the reservoir 40 so that the reservoir 40 can be emptied by advancing the piston 44 of the pump 41.

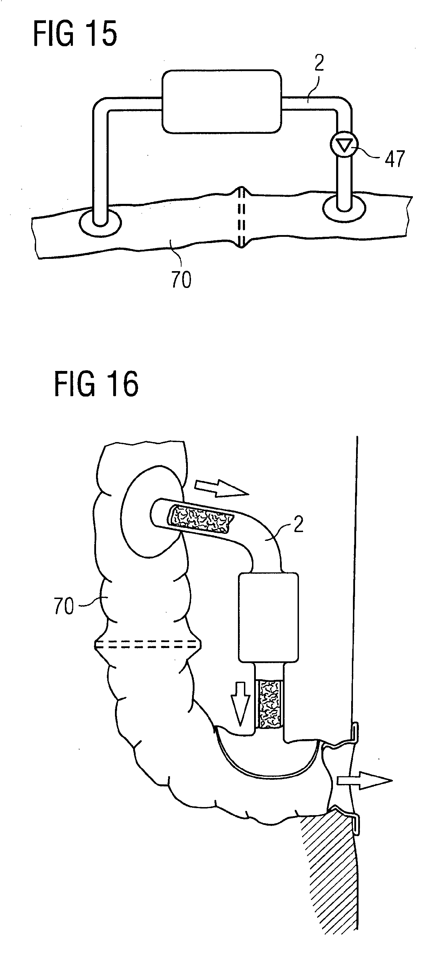

Figure 15 shows an embodiment schematically, wherein the artificial intestine section 2 by-passes a section of the patient's intestine 70, the intestine 70 being closed by sewing so as to direct intestinal content towards the artificial intestine section 2. An exit valve 47 is provided for controlling the flow of intestinal contents from the artificial intestine section 2. The enlarged area of the artificial intestine section represents any kind of element acting on the intestinal contents within the artificial intestine section 2, such as a reservoir, one or more valves, a pump or any other flow control device, possibly including a motor, and the like.
Figure 16 shows a by-passing artificial intestine section 2 in action, further leading to a surgically created stoma. A pump or valve may be contained in the artificial intestine section 2.

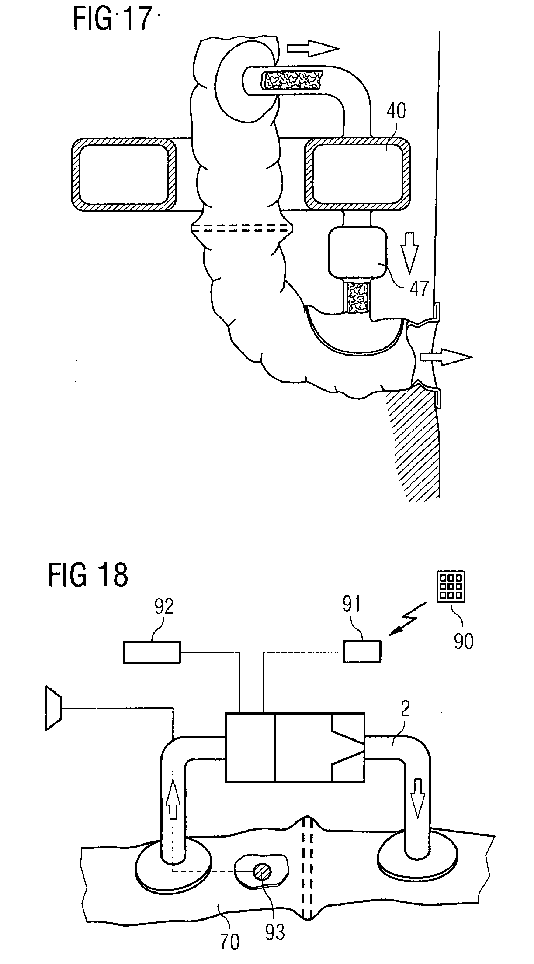

Figure 17 shows the artificial intestine section 2 of Figure 16 with a large reservoir 40 and an exit valve 47 downstream the reservoir 40.
Figure 18 shows the by-passing artificial intestine section 2 including a pump and a valve incorporated therein. Furthermore, a battery 92 implantable in the patient's body and preferably rechargeable provides the artificial intestine section 2 with energy. The artificial intestine section 2 is wirelessly controlled and the battery 92, if rechargeable, wirelessly charged. A sensor 93 implanted on or within the intestine 70 delivers data on the physical conditions within the intestine 70 for controlling the artificial intestine section 2.

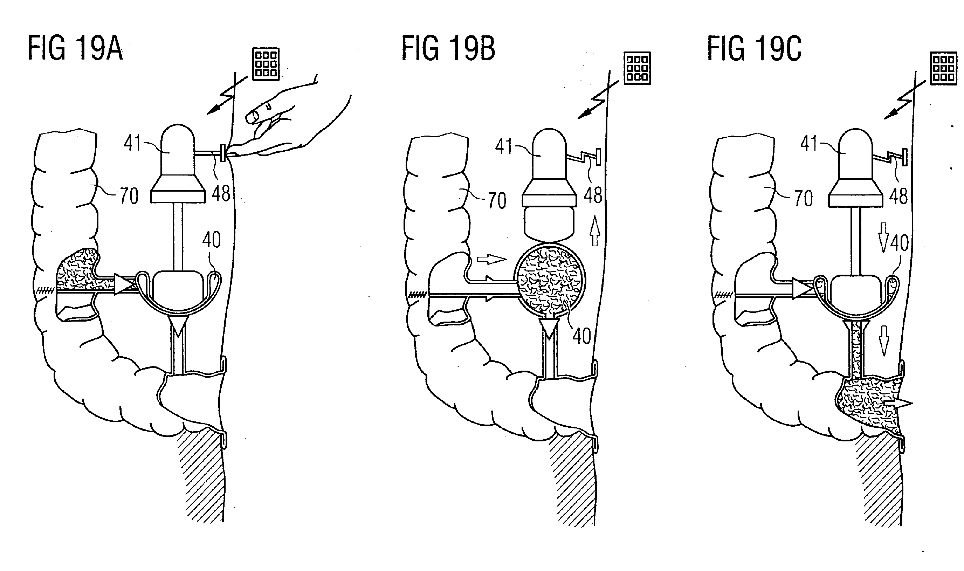

Figures 19A to 19C show an embodiment, where the artificial intestine section 2 comprises a reservoir 40 with a flexible wall. A pump 71 is implanted in the patient's body separate but in close proximity to the reservoir 40 and is used to empty the reservoir 40. The pump 41 is actuated by means of a subcutaneously implanted, manually operable switch 48.

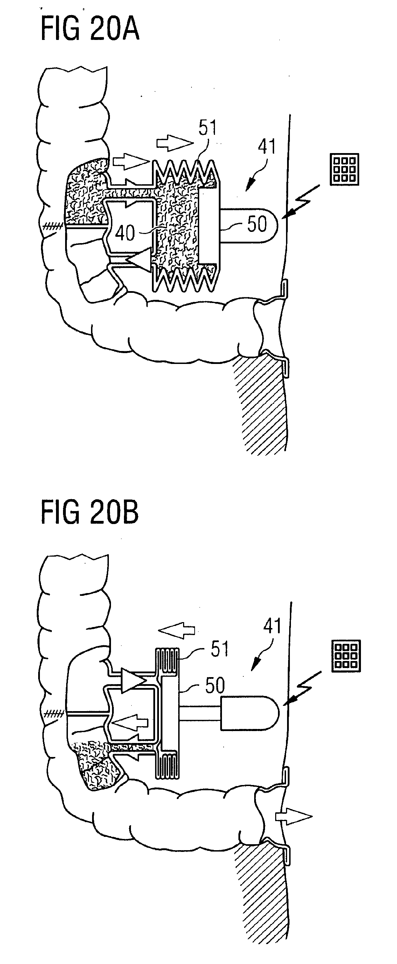

Figures 20A and 20B show a structure similar to the one of Figures 19A to 19C, however, with the pump 41 and the reservoir 40 being fixedly connected to one another. The reservoir 40 is formed by a bellow 51 having an end wall 50 closing the bellow 51 at one end thereof. The end wall 50 makes part of the pump 41 such that a volume of the bellow 51 can be reduced upon advancement of the end wall 50. The bellow 51 is made of a resilient material so as to urge the bellow 51 into a normally extended position.

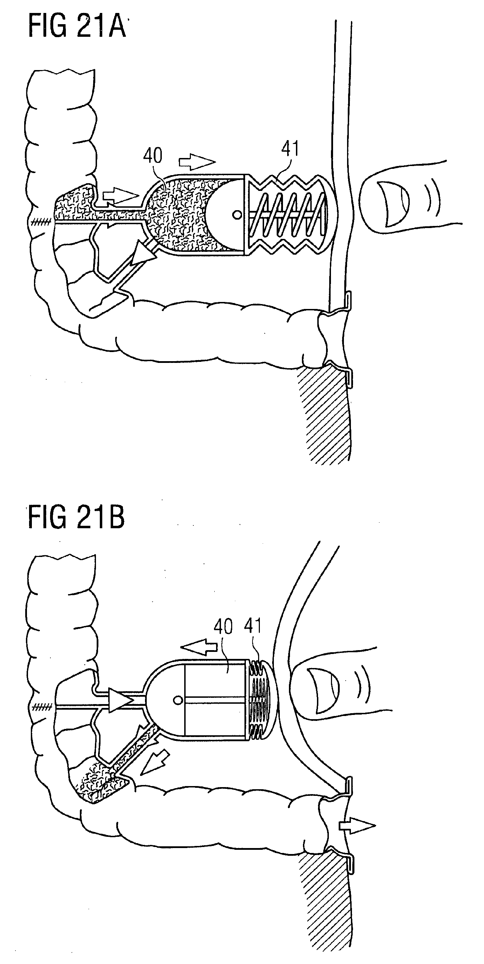

Figures 21A and 21B show a variant to Figures 20A and 20B. Here, the pump 41 and reservoir 40 are integrally combined. The pump 41 is manually operable and subcutaneously mounted so as to be operable from the outside of the patient's body.

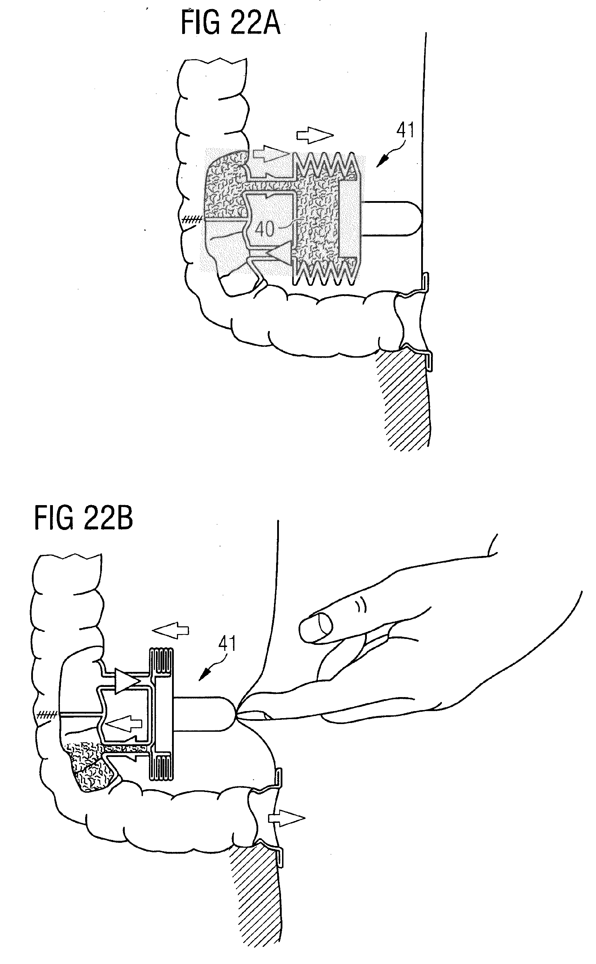

Figures 22A and 22B likewise show a variant to the system shown in Figures 20A and 20B. While in the system of Figures 20A, 20B the pump 41 is automatically driven, such as by an integrated motor, and activated via remote control, the system in Figures 22A and 22B is again manually operable in that the manually operable pump 41 is mounted subcutaneously.

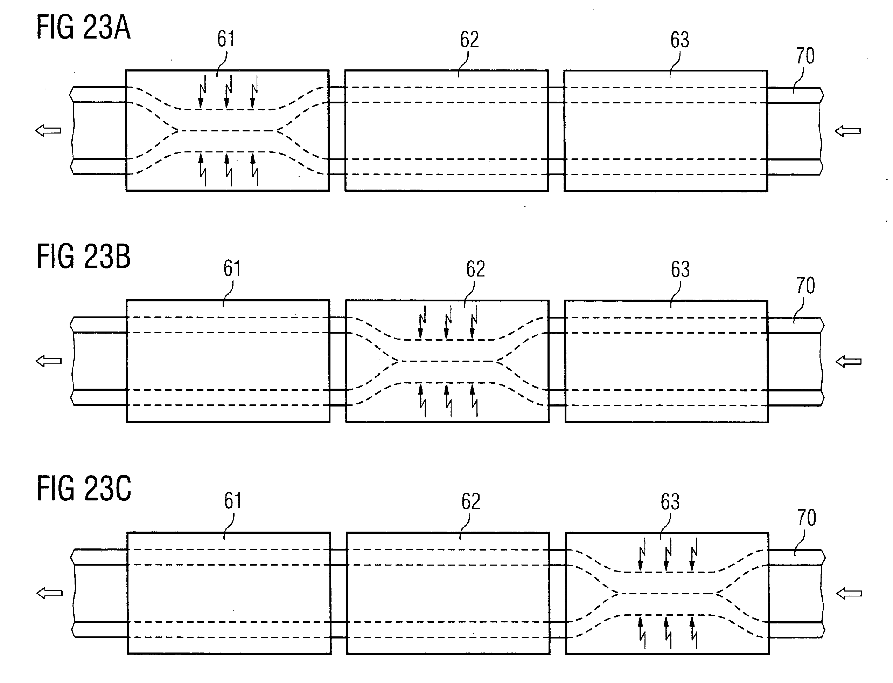

Figures 23A to 23C show a plurality of cooperating valves 61, 62, 63 implanted inside the patient's body and outside the patient's intestine 70. These can be positioned behind and/or in front of the artificial intestine piece along the patient's natural intestine 70. Each of the valves 61, 62, 63 comprises an electrical stimulation device adapted to electrically stimulate muscle or neural tissue of an intestine section so as to cause at least partial contraction of the intestine section. For that purpose, the stimulation device comprises at least one electrode adapted to apply electric pulses to the intestine section. While instead of the three stimulation devices shown, a single stimulation device would be sufficient for opening and closing the intestine, the arrangement of the plurality of stimulation devices is adapted to stimulate different portions of the intestine section over time. The function of the three stimulation devices may also be combined in one integral unit. The direction of natural intestinal contents flow is indicated by arrows. The different portions of the intestine section in a wavelike manner may be made in a direction opposite to the natural intestinal contents flow, as shown in Figures 23A to 23C, so as to close the intestine section. The stimulation in the wavelike manner may also be made in the direction of natural intestinal contents flow to support emptying of the intestine or reservoir.
. Figures 24A to 24C show the stimulation devices of Figures 23A to 23C in combination with constriction devices, such as the bellow valves described in relation to Figures 14A and 14B, for at least partly constricting the intestine section mechanically. Complete constriction is obtained by additional electrical stimulation of the respective intestine sections. The constriction devices may be released in order to allow intestinal contents to flow through.

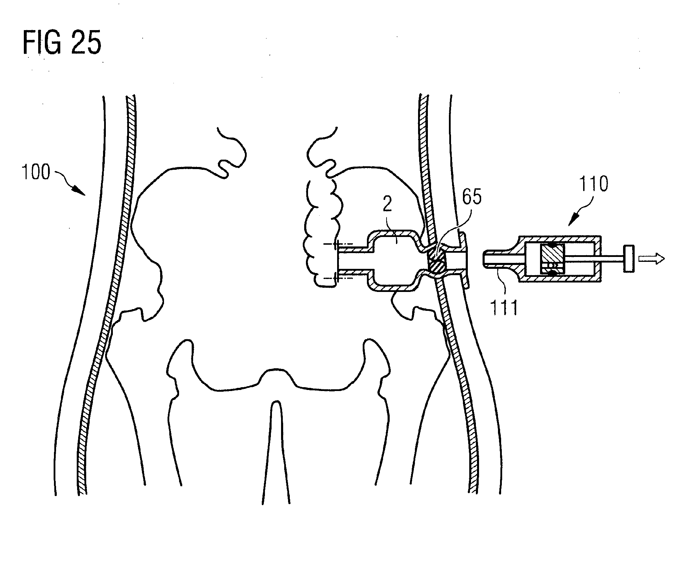

Figure 25 shows a system similar to the system of Figure 1, however, with a flow control device in the form of an exit valve 65 being implanted within the artificial intestine section 2. An external manually driven suction pump 110 is used for emptying the artificial intestine section 2, wherein a conduit 111 on the front end of the pump 110 is inserted from outside the patient's body 100 into the intestine, thereby mechanically urging the exit valve 65 to open.

Preferred aspects of the invention are defined in paragraphs 1 to 179.

### LATERALLY CONNECTED ARTIFICIAL INTESTINE SECTION

1. An artificial intestine section adapted to be implanted inside a patient's body, said intestine section having a first open end portion and a second open end portion in flow communication with one another, and further comprising at least one element adapted to directly or indirectly interact with intestinal contents contained in the artificial intestine section between the first and second open end portions thereof, wherein the at least one element comprises an artificial reservoir between the first and second open end portions for receiving and temporarily collecting therein intestinal contents supplied through the first open end portion, wherein at least the first open end portion is adapted to being connected to a surgically created lateral opening in a wall of the patient's intestine.
2. The artificial intestine section of para 1, wherein the second open end portion is adapted to being connected to a surgically created stomy.
3. The artificial intestine section of para 1, wherein the second open end portion is adapted to being connected to the patient's rectum or anus or to tissue adjacent the patient's anus.
4. The artificial intestine section of para 1, wherein the second open end portion is adapted to being connected to a patient's small intestine.
5. The artificial intestine section of para 1, wherein the second open end portion is adapted to being connected to a patient's large intestine.

### BY-PASS ARRANGEMENT

6. The artificial intestine section of any of paras 1 to 3, wherein both the first and second open end portions are adapted to being connected to a surgically created lateral opening in a wall of the patient's intestine.

### STRUCTURE OF ATTACHMENT

7. The artificial intestine section of any of paras 1 to 6, wherein at least the first open end portion comprises a shoulder portion formed around the end portion for lateral connection to the patient's intestinal wall.
8. The artificial intestine section of para 7, wherein at least a part of the shoulder portion extends laterally from the artificial intestine section by 3 mm to 20 mm.
9. The artificial intestine section of any of paras 7 to 8, wherein the shoulder portion has a curved cross section, so as to generally conform to an intestinal wall when laterally attached thereto.
10. The artificial intestine section of any of paras 7 to 9, wherein the shoulder portion is split into an upper and a lower shoulder portion with a gap between the upper and lower shoulder portions adapted to accommodate intestinal wall tissue therein.
11. The artificial intestine section of para 10, wherein the lower shoulder portion is adapted to being placed inside the patient's intestine through a surgically created lateral opening in the intestinal wall and wherein the upper shoulder portion is adapted to being placed outside the intestinal wall.
12. The artificial intestine section of para 11, wherein the upper shoulder portion and the lower shoulder portion each have a surface area facing the intestinal wall, with the surface area of the upper shoulder portion being larger than the surface area of the lower shoulder portion.
13. The artificial intestine section of any of paras 1 to 12, wherein at least the first open end portion is adapted to being connected to the patient's intestinal wall by gluing.
14. The artificial intestine section of any of paras 1 to 13, wherein at least the first open end portion is adapted to being connected to the patient's intestinal wall by sewing.
15. The artificial intestine section of any of paras 1 to 13, wherein at least the first open end portion is adapted to being connected to the patient's intestinal wall by stapling.
16. The artificial intestine section of any of paras 1 to 14, wherein at least the first open end portion is made from a biocompatible material.
17. The artificial intestine section of para 16, wherein the biocompatible material of the open end portion comprises at least one material of the following group of materials: titanium, stainless steel, ceramics, biocompatible polymer material.
18. The artificial intestine section of para 17, wherein the biocompatible polymer material comprises at least one polymer of the following group of polymers: polytetrafluoroethylene, silicone, polyurethane, expanded polytetrafluoroethylene (ePTFE).
19. The artificial intestine section of any of paras 1 to 18, wherein at least the first open end portion comprises a multilayer material.
20. The artificial intestine section of any of paras 1 to 19, wherein at least the first open end portion comprises a porous ingrowth layer allowing ingrowth of living tissue.
21. The artificial intestine section of para 20, wherein the ingrowth layer has a net-like structure.
22. The artificial intestine section of any of paras 20 to 21, wherein the ingrowth layer is made from Dacron®.

### FLOW CONTROL DEVICE

23. The artificial intestine section of any of paras 1 to 22, wherein the at least one element comprises a flow control device adapted to control flow of intestinal contents from the artificial intestine section through the second open end portion.
24. The artificial intestine section of para 23, wherein the flow control device is adapted to prevent flow of intestinal contents from the artificial intestine section through the second open end portion.

### EXIT VALVE AS FLOW CONTROL DEVICE

25. The artificial intestine section of any of paras 23 to 24, wherein the flow control device comprises at least one valve, including an exit valve preventing intestinal contents flow through the second open end portion in its closed position.
26. The artificial intestine section of para 25, wherein the exit valve is a normally closed valve.

### ENTRY VALVE AS AN ADDITITONAL PART OF THE FLOW CONTROL DEVICE

27. The artificial intestine section of any of paras 25 to 26, wherein the flow control device comprises an entry valve allowing intestinal contents to flow through the first open end portion into the artificial intestine section in its open position.
28. The artificial intestine section of para 27, wherein the entry valve is a normally open valve.
29. The artificial intestine section of any of paras 27 to 28, wherein the exit valve and the entry valve are adapted to cooperate such that when one of the two valves is closed, the other valve is open, and vice versa.

### VALVE TYPES

30. The artificial intestine section of any of paras 25 to 29, wherein the exit valve comprises a central opening which is normally closed by resilient means that can be urged apart mechanically by inserting a conduit through the central opening so as to open the central opening of the exit valve.
31. The artificial intestine section of any of paras 25 to 30, wherein the at least one valve comprises a compartment with a variable volume adapted to open and close the valve by changing the compartment's volume.
32. The artificial intestine section of para 31, wherein the at least one valve comprises at least one passage for filling and emptying the compartment with hydraulic fluid.
33. The artificial intestine section of any of paras 31 to 32, wherein the compartment has at least one flexible wall defining an opening, the opening being adapted to close upon increase of the compartment's volume.
34. The artificial intestine section of any of paras 25 to 30, wherein the at least one valve is a flap valve.
35. The artificial intestine section of para 34, wherein the flap valve comprises a rotatable disc.

### EXTRA VALVE SEPARATE FROM ARTIFICIAL INTESTINE PIECE

36. The artificial intestine section of any of paras 1 to 35, further comprising at least one extra valve adapted to control flow of intestinal contents in a natural section of a patient's intestine upstream and/or downstream the artificial intestine section, wherein the extra valve is adapted to being implanted inside the patient's body outside a section of the patient's natural intestine and comprises at least one element adapted to act on the natural intestine section from the outside thereof so as to prevent intestinal contents flow through the natural intestine section.
37. The artificial intestine section of para 36, wherein the extra valve comprises at least one electrical stimulation device adapted to electrically stimulate muscle or neural tissue of the natural intestine section so as to cause at least partial contraction of the natural intestine section.
38. The artificial intestine section of para 37, wherein the stimulation device comprises at least one electrode adapted to apply electric pulses to the natural intestine section.
39. The artificial intestine section of any of paras 37 to 38, wherein the stimulation device is adapted to stimulate different portions of the natural intestine section over time.
40. The artificial intestine section of para 39, wherein the stimulation device is adapted to stimulate, over time, the different portions of the natural intestine section in a wave like manner in a direction opposite to natural intestinal contents flow.
41. The artificial intestine section of any of paras 36 to 40, wherein the extra valve comprises a constriction device for at least partly constricting the natural intestine section mechanically.
42. The artificial intestine section of para 41, including para 37, wherein the stimulation device is combined with the constriction device so that the stimulation device and the constriction device act on the same natural intestine section.
43. The artificial intestine section of para 42, wherein the constriction device in its normal condition constricts the natural intestine section only partly.
44. The artificial intestine section of any of paras 42 to 43, wherein the stimulation device is adapted to pump intestinal contents along the natural intestine section by, over time, stimulating the different portions of the natural intestine section in a wave like manner in a direction of natural intestinal contents flow.

### PUMP AS PART OF THE IMPLANTABLE FLOW CONTROL DEVICE

45. The artificial intestine section of any of paras 23 to 44, wherein the flow control device comprises a pump for advancing intestinal contents through the second open end portion to outside the artificial intestine section.
46. The artificial intestine section of para 45, wherein the pump is adapted for emptying the reservoir.
47. The artificial intestine section of para 46, wherein the reservoir is formed by a bellow, said bellow having an end wall closing the bellow at one end thereof, said end wall making part of the pump such that a volume of the bellow is reduced upon advancement of said end wall.
48. The artificial intestine section of para 47, wherein the bellow is made of a resilient material so as to urge the bellow into a normally expanded position.
49. The artificial intestine section of para 46, wherein the pump comprises a movable piston, with a front end of the piston extending into the reservoir such that a volume of the reservoir is reduced upon advancement of the piston.
50. The artificial intestine section of para 49, wherein the piston is spring loaded so as to urge the piston into a normally retracted position.
51. The artificial intestine section of para 46, wherein the pump is adapted for being permanently arranged inside the reservoir.
52. The artificial intestine section of para 46, wherein the reservoir has a flexible wall and the pump is adapted for emptying the reservoir by squeezing the reservoir.
53. The artificial intestine section of para 52, wherein the pump includes a constriction device adapted to alternately constrict and release sections of the reservoir so as to pump intestinal contents along the reservoir by, over time, constricting different sections of the reservoir in a wave like manner.
54. The artificial intestine section of para 53, wherein the reservoir has a tube-like form and the pump is a roller pump acting on the tube-like reservoir from the outside thereof.

### MOTOR

55. The artificial intestine section of any of paras 23 to 54, wherein the at least one element comprises at least one motor arranged for automatically driving at least one energy consuming parts of the flow control device.
56. The artificial intestine section of para 55, including the artificial intestine section of any of paras 25 to 44, wherein the at least one motor is arranged for driving at least one of the valve or valves, respectively, between closed and open positions.
57. The artificial intestine section of any of paras 55 to 56, including the artificial intestine section of any of paras 45, to 54, wherein the at least one motor is arranged for driving the pump.
58. The artificial intestine section of any of paras 55 to 57, comprising a manually operable switch for activating the at least one motor, the switch being arranged for subcutaneous implantation so as to be operable from outside the patient's body.
59. The artificial intestine section of any of paras 55 to 58, wherein the motor is arranged to be driven by electric or electromagnetic energy.

### ENERGY SOURCE

60. A system comprising the artificial intestine section of any of paras 1 to 59 and further comprising an energy source for supplying energy directly or indirectly to at least one energy consuming part of the system.
61. The system of para 60, wherein said energy source includes a battery as an energy storage means.
62. The system of para 61, wherein said energy source includes an accumulator as an energy storage means.
63. The system of para 62, wherein the accumulator comprises one or more of a rechargeable battery and a capacitor.
64. The system of any of paras 61 to 63, wherein the energy storage means is adapted for being implanted inside the patient's body.
65. The system of any of paras 61 to 64, wherein the energy storage means supplies energy for at least one energy consuming part of the at least one element.

### WIRELESS ENERGY TRANSMISSION

66. The system of para 60, wherein the energy source comprises a wireless energy transmitter adapted to wirelessly transmit energy from outside the patient's body to the at least one energy consuming part.
67. The system of any of paras 61 to 65, wherein the energy source comprises a wireless energy transmitter adapted to wirelessly transmit energy from outside the patient's body to the energy storage means.
68. The system of para 67, comprising a feedback subsystem adapted to wirelessly send feedback information related to the energy to be stored in the accumulator from inside the human body to the outside thereof, wherein the system is adapted to use the feedback information for adjusting the amount of wireless energy transmitted by the energy transmitter.
69. The system of para 68, wherein the feedback information is related to an energy balance which is defined as the balance between an amount of wireless energy received inside the human body and an amount of energy consumed by the at least one energy consuming part.
70. The system of para 68, wherein the feedback information is related to an energy balance which is defined as the balance between a rate of wireless energy received inside the human body and a rate of energy consumed by the at least one energy consuming part.
71. The system of any of paras 67 to 70, comprising a wireless energy transmitter adapted to wirelessly transmit energy from the energy storage means to the at least one energy consuming part.
72. The system of any of para 66 or 71, wherein the energy consuming part is adapted to directly transform the wirelessly transmitted energy into kinetic energy.
73. The system of any of paras 66 to 71, comprising an implantable energy transforming device for transforming wirelessly transmitted energy into electric energy.
74. The system of para 73, wherein the energy consuming part is driven with the electric energy, as said energy transforming device transforms the wireless energy into the electric energy.

### GALVANIC ENERGY TRANSMISSION

75. The system of any of paras 60 to 65, further comprising galvanic coupling elements between the energy source and the motor for transmitting energy to the motor in contacting fashion.

### CONTROL UNIT

76. A system comprising an artificial intestine piece of any of paras 1 to 59, or the system of any of paras 60 to 75, further comprising a control unit adapted to directly or indirectly control one or more elements of the system.
77. The system of para 76, including para 25, wherein the control unit is adapted to control opening of the exit valve.
78. The system of any of paras 76 to 77, including para 27, wherein the control unit is adapted to control closing of the entry valve.
79. The system of para 78, wherein the control unit is adapted to control opening of the exit valve and closing of the entry valve such that when one of the two valves is closed, the other valve is open, and vice versa.
80. The system of any of paras 76 to 79, including para 45, wherein the control unit is adapted to control actuation of the pump.
81. The system of any of paras 76 to 80, wherein the control unit is operable by the patient.
82. The system of any of paras 76 to 81, wherein at least part of the control unit is implantable in the patient's body.
83. The system of para 82, comprising a manually operable switch for activating the control unit, the switch being arranged for subcutaneous implantation so as to be operable from outside the patient's body.
84. The system of any of paras 76 to 83, wherein the control unit comprises a first part adapted for implantation in the patient's body and a second part adapted to cooperate with the first part from outside the patient's body.
85. The system of para 84, including para 66, wherein the control unit is adapted to transmit data from the second part of the control unit to the implantable first part of the control unit in the same manner as energy is transmitted to the at least one energy consuming part.
86. The system any of paras 84 to 85, wherein the second part of the control unit is adapted to wirelessly transmit a control signal to the implantable first part of the control unit for controlling the at least one energy consuming part from outside the patient's body.
87. The system of any of paras 84 to 86, wherein the implantable first part of the control unit is programmable via the second part of the control unit.
88. The system of any of paras 84 to 87, wherein the implantable first part of the control unit is adapted to transmit a feedback signal to the second part of the control unit.

### SENSOR

89. A system comprising an artificial intestine piece of any of paras 1 to 59, or the system of any of paras 60 to 88, further comprising a physical parameter sensor adapted to directly or indirectly sense a physical parameter of the patient.
90. The system of para 89, wherein the physical parameter sensor is adapted to sense at least one of the following physical parameters of the patient: a pressure within the artificial intestine section, a pressure within the patient's intestine, an expansion of the artificial intestine section, a distension of an intestinal wall of the patient's intestine, a movement of the intestinal wall.
91. A system comprising an artificial intestine piece of any of paras 1 to 59, or the system of any of paras 60 to 90, further comprising a functional parameter sensor adapted to directly or indirectly sense a functional parameter of the system.
92. The system of any of paras 91, wherein the functional parameter sensor is adapted to sense at least one of the following functional parameters of the system: a pressure against a part of the system such as the artificial intestine section, a distension of a part of the system such as a wall of the artificial intestine section, an electrical parameter such as voltage, current or energy balance, a position or movement of a movable part of the system.
93. The system of any of paras 89 to 92, comprising an indicator coupled to the sensor, the indicator being adapted to provide a signal when the sensor senses a value for the parameter beyond a predetermined threshold value.
94. The system of para 93, wherein the signal comprises at least one of the following types of signals: a sound signal, a visual signal.

### INTESTINAL CONTENTS COLLECTING DEVICE (WITH "EXTERNAL" PUMP)

95. A system comprising an artificial intestine piece of any of paras 1 to 59, or the system of any of paras 60 to 94, further comprising an intestinal contents collecting device to be temporarily applied from outside the patient's body.
96. The system of para 95, including para 25, wherein the collecting device comprises a front open end adapted to be applied towards said exit valve so as to provide a flow passage from the exit valve towards the collecting device.
97. The system of para 96, wherein the collecting device front open end is adapted to be applied to said exit valve so as to open the valve and thereby provide the flow passage towards the collecting device.
98. The system of para 97, including para 30, wherein said front open end is adapted to be inserted through the central opening of said exit valve so as to urge apart the resilient means normally closing the central opening.
99. The system of any of paras 95 to 98, wherein the collecting device comprises a suction pump.
100. The system of para 99, wherein the suction pump comprises a piston-cylinder-arrangement.
101. The system of para 100, wherein the suction pump is adapted to be driven manually.
102. The system of any of paras 95 to 101, further comprising a motor connected to the suction pump for driving the pump automatically.
103. The system of any of paras 95 to 102, including any of paras 45 to 54, wherein the suction pump is provided as a back-up pump for a situation where the pump of the flow control device is out of operation.

### METHOD OF TREATMENT (IMPLANTATION)

### LATERAL CONNECTION

104. A surgical method of treating a patient, comprising the steps of:
   - cutting the patient's skin and abdominal wall,
   - dissecting an area of the patient's intestine,
   - surgically creating at least one opening in a wall of the dissected intestinal area so as to create an artificial lateral intestinal opening,
   - providing the artificial intestine section according to any of paras 1 to 59 and affixing the first open end portion to the lateral intestinal opening so as to be in flow communication therewith, and
   - suturing the abdominal wall and skin.
105. A laparoscopic surgical method of treating a patient, comprising the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferably through a second trocar,
- dissecting an area of the intestine,
- surgically creating at least one opening in a wall of the dissected intestinal area so as to create an artificial lateral intestinal opening,
- providing the artificial intestine section according to any of paras 1 to 59 and affixing the first open end portion to the lateral intestinal opening so as to be in flow communication therewith,
- extracting the instruments, camera and trocar, and in relation thereto
- suturing, if necessary, the abdominal wall and permanently closing the skin.

### CLOSING THE INTESTINE BY SEWING

106. The method of any of paras 104 to 105, comprising the steps of:
   - permanently closing the patient's intestine in the dissected portion at a location downstream of the lateral intestinal opening so as to create an upstream part of the intestine including the lateral intestinal opening and a downstream part of the intestine,
   - affixing the second open end portion of the artificial intestine section to the downstream intestinal part.
107. The method of para 106, wherein the step of permanently closing the patient's intestine comprises sewing or stapling, or sewing and stapling, the intestinal wall so as to form a dead end.

### DIVIDING THE INTESTINE

108. The method of any of paras 104 to 105, comprising the steps of:
   - dissecting a portion of the dissected intestinal area downstream of the lateral intestinal opening such that intestinal mesentery connected to the dissected portion is opened in such a way that supply of blood through the mesentery to the dissected intestinal area is maintained as far as possible on both sides of the dissected portion,
   - dividing the patient's intestine in the dissected portion so as to create an upstream part of the intestine with the lateral intestinal opening and a downstream part of the intestine, said downstream intestine part being separate from the upstream intestine part and having a cross-sectional opening at the upstream side thereof, wherein the mesentery maintains a tissue connection between the upstream and downstream intestine parts,
   - affixing the second open end portion of the artificial intestine section to the downstream intestine part, and
   - permanently closing the upstream intestine part at a location downstream of the lateral intestinal opening.

### LATERAL FRONT AND LATERAL END CONNECTION (BY-PASS)

109. The method of any of paras 106 to 107, wherein the step of affixing the second open end portion of the artificial intestine section to the downstream intestinal part comprises the steps of:
   - surgically creating an opening in a wall of the downstream intestinal part at an upstream end thereof so as to create a second artificial lateral intestinal opening, and
   - affixing the second open end portion of the artificial intestine section to the second lateral intestinal opening so as to be in flow communication therewith.
110. The method of para 109, wherein the step of permanently closing the patient's intestine comprises sewing the intestinal wall with two rows of sutures or staples and cutting and dividing the intestine between the sutures or staples so as to form two dead ends.
111. The method of para 108, wherein the step of affixing the second open end portion of the artificial intestine section to the downstream intestinal part comprises the steps of:
   - surgically creating an opening in a wall of the downstream intestinal part at an upstream end thereof so as to create a second artificial lateral intestinal opening,
   - affixing the second open end portion of the artificial intestine section to the second lateral intestinal opening so as to be in flow communication therewith, and
   - permanently closing the cross-sectional opening at the upstream side of the downstream intestine part at a location upstream of the second lateral intestinal opening.

### LATERAL FRONT CONNECTION AND STRAIGHT END CONNECTION

112. The method of para 108, wherein the step of affixing the second open end portion of the artificial intestine section to the downstream intestinal part comprises the step of:
   - affixing the second open end portion of the artificial intestine section to the cross-sectional upstream opening of the downstream intestine part so as to be in flow communication therewith.

### SLEEVE/BULGE CONNECTOR

113. The method of para 112, wherein the step of affixing the second open end portion of the artificial intestine section to the cross-sectional upstream opening of the downstream intestine part comprises:
   - inserting the second open end portion of the artificial intestine section into the upstream opening of the downstream intestine part, and
   - placing a flexible sleeve so as to extend over both the downstream intestine part and second open end portion of the artificial intestine section such that the downstream intestine part is located intermediate the sleeve and the outer surface of the artificial intestine section.
114. The method of para 113, wherein the flexible sleeve is mounted on the outer surface of the second open end portion of the artificial intestine piece so as to be foldable upon itself and wherein the step of placing the flexible sleeve so as to extend over both the downstream intestine part and second open end portion of the artificial intestine section comprises folding the flexible sleeve upon itself such that the downstream intestine part is located intermediate the folded sleeve.
115. The method of para 112, wherein the step of affixing the second open end portion of the artificial intestine section to the cross-sectional upstream opening of the downstream intestine part comprises:
   - inserting the artificial intestine section having a bulge formed on the outside thereof into the upstream opening of the downstream intestine part so that the downstream intestine part extends over the bulge from one side of the bulge, and
   - advancing a blocking ring over the downstream intestine part towards the bulge from the respective other side of the bulge such that the downstream intestine part is located intermediate the outer surface of the artificial intestine section and the blocking ring.

### EXIT THROUGH STOMA

116. The method of any of paras 104 to 115, further comprising the steps of:
   - cutting the patient's skin and abdominal wall so as to create an opening for an intestinal stomy,
   - dissecting the area of the stomy opening,
   - dividing the intestine at a location downstream of the artificial intestine piece so as to create an upstream natural intestine section having a cross-sectional opening at the downstream end thereof and a downstream natural intestine section,
   - dissecting the mesentery of the upstream natural intestine section in the area of the cross-sectional opening thereof to prepare for creating the intestinal stomy,
   - advancing the downstream end of the upstream natural intestine section through the abdominal wall and skin, and
   - suturing the cross-sectional opening of the upstream natural intestine section to the skin with the intestinal mucosa turned inside out, thereby achieving the intestinal stomy.

### EXIT THROUGH ANUS

117. The method of any of paras 104 to 115, further comprising the steps of:
   - dividing the intestine at a location downstream of the artificial intestine piece so as to create an upstream natural intestine section having a cross-sectional opening at the downstream end thereof and a downstream natural intestine section leading to the patient's anus,
   - dissecting the area of the patient's anus and surgically separating the downstream natural intestine section from the patient's anus, whereas the steps of dividing the intestine and separating the intestine section leading to the patient's anus can alternatively be carried out in reversed order,
   - dissecting the mesentery of the upstream natural intestine section in the area of the cross-sectional opening at the downstream end thereof to prepare for connecting the upstream natural intestine section to the patient's anus or tissue adjacent the patient's anus,
   - advancing the downstream end of the upstream natural intestine section through the patient's anus, and
   - suturing the cross-sectional opening of the upstream natural intestine section to the patient's anus or tissue adjacent the patient's anus.
118. The method of any of paras 116 to 117, wherein the natural intestine section is selected from the patient's small intestine.
119. The method of any of paras 116 to 117, wherein the natural intestine section is selected from the patient's large intestine.

### STRUCTURE OF ATTACHMENT

120. The method of any of paras 104 to 119, wherein the step of affixing the first open end portion of the artificial intestine section to the lateral intestinal opening so as to be in flow communication therewith comprises connecting a shoulder portion, which is formed around the first open end portion of the artificial intestine section, to the patient's intestinal wall so as to surround the lateral intestinal opening.
121. The method of para 120, wherein the step of connecting the shoulder portion comprises attaching the shoulder portion to the patient's outer intestinal wall.
122. The method of para 120, wherein the shoulder portion is split into an upper and a lower shoulder portion with a gap between the upper and lower shoulder portions, and wherein the step of affixing the first open end portion of the artificial intestine section to the lateral intestinal opening comprises placing the lower shoulder portion inside the patient's intestine and the upper shoulder portion outside the patient's intestine such that intestinal wall tissue is accommodated in the gap.
123. The method of any of paras 120 to 122, wherein the step of affixing the first open end portion of the artificial intestine section to the lateral intestinal opening comprises gluing the first open end portion to the patient's intestinal wall.
124. The method of any of paras 120 to 123, wherein the step of affixing the first open end portion of the artificial intestine section to the lateral intestinal opening comprises sewing the first open end portion to the patient's intestinal wall.
125. The method of any of paras 120 to 124, wherein the step of affixing the first open end portion of the artificial intestine section to the lateral intestinal opening comprises stapling the first open end portion to the patient's intestinal wall.
126. The method of any of paras 120 to 125, comprising the step of selecting for the first open end portion at least one biocompatible material from the following group of materials: titanium, stainless steel, ceramics, biocompatible polymer material.
127. The method of para 126, comprising the step of selecting for the first open end at least one biocompatible polymer from the following group of polymers: polytetrafluoroethylene, silicone, polyurethane, expanded polytetrafluoroethylene (ePTFE).
128. The method of any of paras 120 to 127, comprising the step of selecting for the first open end a multilayer material.
129. The method of any of paras 120 to 128, comprising the step of providing the first open end portion with a porous ingrowth layer allowing ingrowth of living tissue.
130. The method of para 129, comprising the step of choosing the ingrowth layer to have a net-like structure.

### BLACK BOX WITHIN ARTIFICIAL INTESTINE PIECE

131. The method of any of paras 104 to 130, comprising the step of implanting along with the artificial intestine section at least one element adapted to directly or indirectly interact with intestinal contents contained in the artificial intestine section between the first and second open end portions thereof.

### RESERVOIR

132. The method of para 131, wherein the at least one element comprises an artificial reservoir between the first and second open end portions for receiving and temporarily collecting therein intestinal contents supplied through the first open end portion.

### FLOW CONTROL DEVICE

133. The method of any of paras 131 to 132, wherein the at least one element comprises a flow control device adapted to control flow of intestinal contents from the artificial intestine section through the second open end portion.

### EXIT VALVE

134. The method of para 133, wherein the flow control device comprises an exit valve preventing intestinal contents flow through the second open end portion in its closed position.

### ENTRY VALVE

135. The method of any of paras 133 to 134, wherein the flow control device comprises an entry valve allowing intestinal contents to flow through the first open end portion into the artificial intestine section in its open position.

### PUMP

136. The method of any of paras 133 to 135, wherein the flow control device comprises a pump for advancing intestinal contents through the second open end portion to outside the artificial intestine section.
137. The method of para 136, wherein the pump comprises a manually operable switch for activating the pump, the method further comprising the step of implanting the switch subcutaneously so as to be operable from outside the patient's body.

### MOTOR

138. The method of any of paras 133 to 136, wherein the at least one element comprises at least one motor arranged for automatically driving one or more energy consuming part of the flow control device.
139. The method of para 138, wherein the motor comprises a manually operable switch for activating the motor, the method further comprising the step of implanting the switch subcutaneously so as to be operable from outside the patient's body.

### ENERGY SOURCE / ENERGY TRANSMISSION

140. The method of any of paras 104 to 139, further comprising the step of implanting inside the patient's body an energy source for supplying at least one energy consuming part with energy.
141. The method of para 140, wherein the energy source comprises energy storage means.
142. The method of any of paras 140 to 141, further comprising the step of implanting an energy transforming device for transforming wireless energy into electric energy.
143. The method of any of paras 140 to 142, further comprising the step of implanting galvanic coupling elements for transmitting energy to the energy consuming part in contacting fashion.

### CONTROL UNIT

144. The method of any of paras 104 to 143, further comprising the step of implanting inside the patient's body at least a part of a control unit adapted to directly or indirectly control one or more elements implanted in the patient's body.
145. The method of para 144, wherein the control unit comprises a manually operable switch for activating the control unit, the method further comprising the step of implanting said switch subcutaneously so as to be operable from outside the patient's body.

### SENSOR

146. The method of any of paras 104 to 145, further comprising the step of implanting a physical parameter sensor adapted to directly or indirectly sense a physical parameter inside the patient.
147. The method of any of paras 104 to 146, further comprising the step of implanting a functional parameter sensor adapted to directly or indirectly sense a functional parameter of the system inside the patient.
148. The method of any of paras 146 to 147, wherein the sensor is a pressure sensor and is placed in the artificial intestine section or the patient's natural intestine so as to sense the pressure within the artificial intestine section or patient's natural intestine, respectively.
149. The method of any of paras 146 to 148, wherein the sensor is a tension sensor and is placed in contact with the artificial intestine section or the patient's intestine so as to sense an expansion of the artificial intestine section or patient's natural intestine, respectively.
150. The method of any of paras 146 to 149, wherein the sensor is a movement sensor and is placed in contact with the artificial intestine section or the patient's natural intestine so as to sense movement of the artificial intestine section or patient's natural intestine, respectively.
151. The method of any of paras 147 to 150, wherein the sensor is adapted to measure at least one of the following functional parameters: an electrical parameter such as voltage, current or energy balance or a stimulation parameter in relation to the system.

### USE

152. A method of treating a patient by means of the artificial intestine section of para 1, comprising the step of actuating the at least one element so as to interact with intestinal contents contained in the artificial intestine section between the first and second open end portions thereof.

### EXIT AND ENTRY VALVE

153. The method of treating a patient according to para 152, wherein the at least one element comprises an exit valve preventing intestinal contents flow from the artificial intestine section through the second open end portion in its closed position, the method further comprising the steps of opening the exit valve and then removing intestinal contents from the artificial intestine section.
154. The method of treating a patient according to para 153, wherein the at least one element comprises an entry valve allowing intestinal contents to flow through the first open end portion into the artificial intestine section in its open position, the method further comprising the step of closing the entry valve before removing intestinal contents from the artificial intestine section.
155. The method of treating a patient according to any of paras 153 to 154, comprising the step of inserting a conduit from outside the patient's body into the artificial intestine section, thereby mechanically urging the exit valve to open.

### PUMP

156. The method of any of paras 152 to 155, wherein the at least one element comprises a pump, the method further comprising the step of advancing intestinal contents from the artificial intestine section through the second open end portion thereof to outside the artificial intestine section by means of the pump.
157. The method of para 156, further comprising the step of activating the pump by manually operating a subcutaneously arranged actuator from outside the patient's body.
158. The method of any of paras 152 to 155, further comprising the steps of providing a flow passage so as to extend from the artificial intestine section to an external collecting device and removing intestinal contents from the artificial intestine section by means of a suction pump.

### MOTOR

159. The method of para 158, comprising the step of driving the suction pump by means of a motor.
160. The method of any of paras 153 to 155, wherein the at least one element comprises a motor, the method further comprising the step of driving at least the exit valve between its closed and open positions by means of the motor.
161. The method of para 156, wherein the at least one element comprises a motor, the method further comprising the step of driving at least the pump by means of the motor.
162. The method of any of paras 160 to 161, further comprising the step of activating the motor by manually operating a subcutaneously arranged actuator from outside the patient's body.

### ENERGY

163. The method of any of paras 152 to 162, further comprising the step of transmitting energy from outside the patient's body to at least one implanted energy consuming part.
164. The method of para 163, wherein in the step of transmitting energy from outside the patient's body to the energy consuming part, the energy is transmitted wirelessly.
165. The method of any of paras 152 to 162, further comprising the steps of:
   - transmitting energy wirelessly,
   - transforming the wirelessly transmitted energy into electric energy by means of an energy transforming device,
   - storing the transformed energy in an energy storage means, and
   - supplying the stored energy from the energy storage means to at least one implanted energy consuming part.
166. The method of para 165, wherein in the step of supplying the energy from the storage means to the energy consuming part, the energy is transmitted wirelessly.
167. The method of any of paras 165 to 166, comprising the step of transforming at least part of the wirelessly transmitted energy into electric energy and supplying said part of transformed energy to the energy consuming part, as said part of the wirelessly transmitted energy is transformed into the electric energy.

### CONTROL

168. The method of any of paras 152 to 167, wherein a first part of a control unit for controlling at least one implanted energy consuming part is implanted inside the patient's body, the method further comprising the step of using an external second part of the control unit adapted to cooperate with the first part from outside the patient's body to transmit data to the implanted first part of the control
169. The method of para 168, including para 163, wherein the data are transmitted to the implanted first part of the control unit in the same manner as energy is transmitted for driving the implanted energy consuming part.
170. The method of any of paras 168 to 169, wherein the data are transmitted wirelessly to the implanted first part of the control unit.
171. The method of any of paras 168 to 170, wherein the data are transmitted wirelessly via a wireless control signal.
172. The method of any of paras 168 to 171, comprising the step of programming the implanted first part of the control unit via the external second part of the control unit.
173. The method of any of paras 168 to 172, comprising the step of transmitting a feedback signal from the implanted first part of the control unit to the external second part of the control unit.

### SENSOR

174. The method of any of paras 152 to 173, comprising the step of sensing a physical parameter in the patient's body.
175. The method of para 174, wherein the step of sensing a physical parameter in the patient's body comprises sensing at least one of the following physical parameters of the patient: a pressure within the artificial intestine section, a pressure within the patient's natural intestine, an expansion of the artificial intestine section, a distension of an intestinal wall of the patient's natural intestine, a movement of the patient's intestinal wall.
176. The method of any of paras 152 to 175, comprising the step of sensing a functional parameter of the system in the patient's body.
177. The method of para 176, wherein the step of sensing a functional parameter of the system in the patient's body comprises sensing at least one of the following parameters: a pressure against a part of the system such as the artificial intestine section, a distension of a part of the system such as a wall of the artificial intestine section, an electrical parameter such as voltage, current or energy balance, a position or movement of a movable part of the system.
178. The method of any of paras 174 to 177, comprising the step of providing a signal when a value for the physical parameter sensed is beyond a predetermined threshold value.
179. The method of para 178, wherein the step of providing a signal includes at least one of the following types of signals: a sound signal, a visual signal.

## Claims

1. An artificial intestine section (2) adapted to be implanted inside a patient's body (100), said intestine section (2) having a first open end portion (3) and a second open end portion (4) in flow communication with one another, and further comprising at least one element adapted to directly or indirectly interact with intestinal contents contained in the artificial intestine section (2) between the first and second open end portions (3, 4) thereof, wherein the at least one element comprises an artificial reservoir (40) between the first and second open end portions (3, 4) for receiving and temporarily collecting therein intestinal contents supplied through the first open end portion (3), **characterized in that** at least the first open end portion (3) is adapted to being connected to a surgically created lateral opening in a wall of the patient's intestine (70, 80).

2. The artificial intestine section (2) of claim 1, wherein the second open end portion (4) is adapted to being connected to a surgically created stomy (170) or to the patient's rectum or anus or to tissue adjacent the patient's anus.

3. The artificial intestine section (2) of claim 1, wherein the second open end portion (4) is adapted to being connected to a patient's small intestine (70) or to a patient's large intestine (80).

4. The artificial intestine section (2) of any of claims 1 to 3, wherein both the first and second open end portions (3, 4) are adapted to being connected to a surgically created lateral opening in a wall of the patient's intestine (70, 80), or wherein the second open end portion (4) is adapted to being connected to a cross-sectional opening of the patient's intestine (70, 80).

5. The artificial intestine section (2) of any of claims 1 to 4, wherein at least the first open end portion (3) comprises a shoulder portion (23) formed around the end portion for lateral connection to the patient's intestinal wall, wherein at least a part of the shoulder portion (23) extends laterally from the artificial intestine section (2) by 3 mm to 20 mm, wherein the shoulder portion (23) has a curved cross section, so as to generally conform to an intestinal wall when laterally attached thereto, wherein the shoulder portion (23) is split into an upper (24) and a lower shoulder portion (25) with a gap (26) between the upper (24) and lower shoulder portions (25) adapted to accommodate intestinal wall tissue therein, wherein the lower shoulder portion (25) is adapted to being placed inside the patient's intestine (70, 80) through a surgically created lateral opening (72) in the intestinal wall and wherein the upper shoulder portion (24) is adapted to being placed outside the intestinal wall.

6. The artificial intestine section (2) of any of claims 1 to 5, wherein at least the first open end portion (3) is adapted to being connected to the patient's intestinal wall by gluing, sewing or stapling.

7. The artificial intestine section (2) of any of claims 1 to 6, wherein at least the first open end portion (3) is made from a biocompatible material, wherein the biocompatible material of the open end portion (3) comprises at least one material of the following group of materials: titanium, stainless steel, ceramics, biocompatible polymer material, wherein the biocompatible polymer material comprises at least one polymer of the following group of polymers: polytetrafluoroethylene, silicone, polyurethane, expanded polytetrafluoroethylene (ePTFE).

8. The artificial intestine section (2) of any of claims 1 to 7, wherein at least the first open end portion (3) comprises a multilayer material.

9. The artificial intestine section (2) of any of claims 1 to 8, wherein at least the first open end portion (3) comprises a porous ingrowth layer allowing ingrowth of living tissue.

10. The artificial intestine section (2) of any of claims 1 to 9, comprising a flexible sleeve (10) adapted to extend over both the second open end portion (4) and a cross-sectional opening of the patient's intestine (70, 80) such that part of the intestine (70, 80) is located intermediate the sleeve (10) and an outer surface (6) of the second open end portion (4), wherein the sleeve (10) serves to improve the strength of the connection against axial forces resulting from peristaltic movements of an intestine (70, 80) which tend to pull on the intestine (70, 80), when implanted.

11. The artificial intestine section (2) of claim 10, wherein said flexible sleeve (10) is mounted on an outer surface (6) of the second open end portion (4) so as to be foldable upon itself such that the patient's intestine (70, 80) can be located intermediate the folded sleeve (10).

12. The artificial intestine section (2) of claim 10 or 11, wherein the sleeve (10) is rolled upon itself and can be unrolled such that the part (81) of the intestine (80) is located intermediate the sleeve (10) and the outer surface (6) of the second open end portion (4).

13. The artificial intestine section (2) of any of claims 1 to 12, comprising:
- a bulge (15) formed on the outer surface of the second open end portion (4) for a part of the patient's intestine (70, 80) having a cross-sectional opening to be extended over the bulge (15) from one side of the bulge (15), and
- a blocking ring (30) adapted to be advanced over the intestine part from the respective other side of the bulge (15) such that the intestine part is located intermediate the outer surface of the artificial intestine piece and the blocking ring (30).

14. The artificial intestine section (2) of any of claims 1 to 13, wherein the at least one element comprises a flow control device adapted to control flow of intestinal contents from the artificial intestine section (2) through the second open end portion (4), wherein the flow control device is adapted to prevent flow of intestinal contents from the artificial intestine section (2) through the second open end portion (4).

15. The artificial intestine section (2) of claim 14, wherein the flow control device comprises at least one valve, including an exit valve (43) preventing intestinal contents flow through the second open end portion (4) in its closed position.

16. The artificial intestine section (2) of any of claims 14 or 15, wherein the flow control device comprises an entry valve (42) allowing intestinal contents to flow through the first open end portion (3) into the artificial intestine section (2) in its open position, wherein the artificial intestine section (2) further comprises a control unit (90, 91) for controlling the exit valve (43) and the entry valve (42) such that when one of the two valves (42, 43) is closed, the other valve (43, 42) is open, and vice versa.

17. The artificial intestine section (2) of any of claims 15 or 16, wherein the at least one valve comprises a compartment with a variable volume adapted to open and close the valve by changing the compartment's volume.

18. The artificial intestine section (2) of any of claims 1 to 17, further comprising at least one extra valve adapted to control flow of intestinal contents in a natural section of a patient's intestine (70, 80) upstream and/or downstream the artificial intestine section (2), wherein the extra valve is adapted to being implanted inside the patient's body (100) outside a section of the patient's natural intestine (70, 80) and comprises at least one element adapted to act on the natural intestine section (70, 80) from the outside thereof so as to prevent intestinal contents flow through the natural intestine section (70, 80).

19. The artificial intestine section (2) of any of claims 14 to 18, wherein the flow control device comprises a pump (41) for advancing intestinal contents through the second open end portion (4) to outside the artificial intestine section (2) and/or wherein the at least one element comprises at least one motor arranged for automatically driving at least one energy consuming part of the flow control device.

20. A system comprising the artificial intestine section (2) of any of claims 1 to 19 and further comprising an energy source for supplying energy directly or indirectly to at least one energy consuming part of the system.

21. The system of claim 20, wherein the energy source comprises a wireless energy transmitter adapted to wirelessly transmit energy from outside the patient's body (100) to the at least one energy consuming part and further comprising a feedback subsystem adapted to wirelessly send feedback information related to the energy to be stored in the accumulator from inside the human body to the outside thereof, wherein the system is adapted to use the feedback information for adjusting the amount of wireless energy transmitted by the energy transmitter.

22. The system of claim 21, wherein the feedback information is related to an energy balance which is defined as the balance between an amount of wireless energy received inside the human body and an amount of energy consumed by the at least one energy consuming part, or wherein the feedback information is related to an energy balance which is defined as the balance between a rate of wireless energy received inside the human body and a rate of energy consumed by the at least one energy consuming part.

23. The system of any of claims 21 or 22, comprising an implantable energy transforming device for transforming wirelessly transmitted energy into electric energy.

24. A system comprising an artificial intestine (2) piece of any of claims 1 to 19, or the system of any of claims 20 to 23, further comprising a control unit (90, 91) adapted to directly or indirectly control one or more elements of the system.

25. The system of claim 24, comprising a manually operable switch (48) for activating the control unit (90, 91), the switch (48) preferably being arranged for subcutaneous implantation so as to be operable from outside the patient's body (100), wherein the control unit (90, 91) comprises a first part (91) adapted for implantation in the patient's body (100) and a second part (90) adapted to cooperate with the first part (91) from outside the patient's body (100), wherein the second part (90) of the control unit (90, 91) is adapted to wirelessly transmit a control signal to the implantable first part (91) of the control unit (90, 91) for controlling the at least one energy consuming part from outside the patient's body (100).

26. A system comprising an artificial intestine (2) piece of any of claims 1 to 19, or the system of any of claims 20 to 25, further comprising a physical parameter sensor adapted to directly or indirectly sense a physical parameter of the patient, wherein the physical parameter sensor is adapted to sense at least one of the following physical parameters of the patient: a pressure within the artificial intestine section (2), a pressure within the patient's intestine (70, 80), an expansion of the artificial intestine section (2), a distension of an intestinal wall of the patient's intestine (70, 80), a movement of the intestinal wall and/or further comprising a functional parameter sensor adapted to directly or indirectly sense a functional parameter of the system, wherein the functional parameter sensor is adapted to sense at least one of the following functional parameters of the system: a pressure against a part of the system such as the artificial intestine section (2), a distension of a part of the system such as a wall of the artificial intestine section (2), an electrical parameter such as voltage, current or energy balance, a position or movement of a movable part of the system.

## Patentansprüche

1. Künstlicher Darmabschnitt (2), der angepasst ist, in einen Körper (100) eines Patienten implantiert zu werden, wobei der Darmabschnitt (2) einen ersten offenen Endteil (3) und einen zweiten offenen Endteil (4) in Flusskommunikation miteinander aufweist, und des Weiteren zumindest ein Element umfasst, das angepasst ist, mit in dem künstlichen Darmabschnitt (2) zwischen dem ersten und zweiten offenen Endteil (3, 4) davon enthaltenen Darminhalten direkt oder indirekt zu interagieren, wobei das zumindest eine Element ein künstliches Reservoir (40) zwischen dem ersten und zweiten offenen Endteil (3, 4) zum Aufnehmen und temporären Sammeln von durch den ersten offenen Endteil (3) gelieferten Darminhalten darin umfasst, **dadurch gekennzeichnet, dass** zumindest der erste offene Endteil (3) angepasst ist, mit einer chirurgisch hergestellten seitlichen Öffnung in einer Wand des Darms (70, 80) des Patienten verbunden zu werden.

2. Künstlicher Darmabschnitt (2) nach Anspruch 1, wobei der zweite offene Endteil (4) angepasst ist, mit einem chirurgisch hergestellten Stoma (170) oder mit dem Rektum oder Anus des Patienten oder mit Gewebe benachbart zum Anus des Patienten verbunden zu werden.

3. Künstlicher Darmabschnitt (2) nach Anspruch 1, wobei der zweite offene Endteil (4) angepasst ist, mit dem Dünndarm (70) eines Patienten oder dem Dickdarm (80) eines Patienten verbunden zu werden.

4. Künstlicher Darmabschnitt (2) nach einem der Ansprüche 1 bis 3, wobei sowohl der erste als auch zweite offene Endteil (3, 4) angepasst sind, mit einer chirurgisch hergestellten seitlichen Öffnung in einer Wand des Darms (70, 80) des Patienten verbunden zu werden, oder wobei der zweite offene Endteil (4) angepasst ist, mit einer Querschnittsöffnung des Darms (70, 80) des Patienten verbunden zu werden.

5. Künstlicher Darmabschnitt (2) nach einem der Ansprüche 1 bis 4, wobei zumindest der erste offene Endteil (3) einen Schulterteil (23) umfasst, der um den Endteil herum zur seitlichen Verbindung mit der Darmwand des Patienten gebildet ist, wobei sich zumindest ein Teil des Schulterteils (23) seitlich von dem künstlichen Darmabschnitt (2) um 3 mm bis 20 mm erstreckt, wobei der Schulterteil (23) einen gekrümmten Querschnitt aufweist, um im Allgemeinen einer Darmwand zu entsprechen, wenn er seitlich daran angebracht ist, wobei der Schulterteil (23) in einen oberen (24) und einen unteren Schulterteil (25) unterteilt ist, wobei eine Lücke (26) zwischen dem oberen (24) und dem unteren Schulterteil (25) angepasst ist, Darmwandgewebe darin aufzunehmen, wobei der untere Schulterteil (25) angepasst, durch eine chirurgisch hergestellte seitliche Öffnung (72) in der Darmwand innerhalb des Darms (70, 80) des Patienten platziert zu werden, und wobei der obere Schulterteil (24) angepasst ist, außerhalb der Darmwand platziert zu werden.

6. Künstlicher Darmabschnitt (2) nach einem der Ansprüche 1 bis 5, wobei zumindest der erste offene Endteil (3) angepasst ist, mit der Darmwand des Patienten durch Kleben, Nähen oder Klammern verbunden zu werden.

7. Künstlicher Darmabschnitt (2) nach einem der Ansprüche 1 bis 6, wobei zumindest der erste offene Endteil (3) aus einem biokompatiblen Material besteht, wobei das biokompatible Material des offenen Endteils (3) zumindest ein Material aus der folgenden Gruppe von Materialien umfasst: Titan, Edelstahl, Keramik, biokompatibles Polymermaterial, wobei das biokompatible Polymermaterial zumindest ein Polymer aus der folgenden Gruppe von Polymeren umfasst: Polytetrafluorethylen, Silikon, Polyurethan, expandiertes Polytetrafluorethylen (ePTFE).

8. Künstlicher Darmabschnitt (2) nach einem der Ansprüche 1 bis 7, wobei zumindest der erste offene Endteil (3) ein Mehrschichtmaterial umfasst.

9. Künstlicher Darmabschnitt (2) nach einem der Ansprüche 1 bis 8, wobei zumindest der erste offene Endteil (3) eine poröse Einwachsschicht umfasst, die ein Einwachsen von lebendem Gewebe erlaubt.

10. Künstlicher Darmabschnitt (2) nach einem der Ansprüche 1 bis 9, umfassend eine flexible Manschette (10), die angepasst ist, sich sowohl über den zweiten offenen Endteil (4) als auch eine Querschnittsöffnung des Darms (70, 80) des Patienten zu erstrecken, so dass sich ein Teil des Darms (70, 80) zwischen der Manschette (10) und einer äußeren Oberfläche (6) des zweiten offenen Endteils (4) befindet, wobei die Manschette (10), wenn implantiert, dazu dient, die Stärke der Verbindung gegen axiale Kräfte zu verstärken, die von peristaltischen Bewegungen eines Darms (70, 80) her resultieren, die dazu neigen, am Darm (70, 80) zu ziehen

11. Künstlicher Darmabschnitt (2) nach Anspruch 10, wobei die flexible Manschette (10) auf einer äußeren Oberfläche (6) des zweiten offenen Endteils (4) angebracht ist, um um sich selbst faltbar zu sein, so dass sich der Darm (70, 80) des Patienten zwischen der gefalteten Manschette (10) befinden kann.

12. Künstlicher Darmabschnitt (2) nach Anspruch 10 oder 11, wobei die Manschette (10) um sich selbst aufgerollt ist und abgerollt werden kann, so dass sich der Teil (81) des Darms (80) zwischen der Manschette (10) und der äußeren Oberfläche (6) des zweiten offenen Endteils (4) befindet.

13. Künstlicher Darmabschnitt (2) nach einem der Ansprüche 1 bis 12, umfassend:
- eine Ausbuchtung (15), die auf der äußeren Oberfläche des zweiten offenen Endteils (4) gebildet ist, damit sich ein Teil des Darms (70, 80) des Patienten mit einer Querschnittöffnung über die Ausbuchtung (15) von einer Seite der Ausbuchtung (15) erstreckt, und
- einen Blockierring (30), der angepasst ist, über den Darmteil von der entsprechenden anderen Seite der Ausbuchtung (15) vorgeschoben zu werden, so dass sich der Darmteil zwischen der äußeren Oberfläche des künstlichen Darmstücks und dem Blockierring (30) befindet.

14. Künstlicher Darmabschnitt (2) nach einem der Ansprüche 1 bis 13, wobei das zumindest eine Element eine Flusssteuervorrichtung umfasst, die angepasst ist, einen Fluss von Darminhalten von dem künstlichen Darmabschnitt (2) durch den zweiten offenen Endteil (4) zu steuern, wobei die Flusssteuervorrichtung angepasst ist, einen Fluss von Darminhalten von dem künstlichen Darmabschnitt (2) durch den zweiten offenen Endteil (4) zu verhindern.

15. Künstlicher Darmabschnitt (2) nach Anspruch 14, wobei die Flusssteuervorrichtung zumindest ein Ventil umfasst, einschließlich eines Ausgangsventils (43), das in seiner geschlossenen Position verhindert, dass Darminhalte durch den zweiten offenen Endteil (4) fließen.

16. Künstlicher Darmabschnitt (2) nach einem der Ansprüche 14 oder 15, wobei die Flusssteuervorrichtung ein Eingangsventil (42) umfasst, das in seiner offenen Position erlaubt, dass Darminhalte durch den ersten offenen Endteil (3) in den künstlichen Darmabschnitt (2) hinein fließen, wobei der künstliche Darmabschnitt (2) des Weiteren eine Steuereinheit (90, 91) zum Steuern des Ausgangsventils (43) und des Eingangsventils (42) umfasst, so dass, wenn eines der zwei Ventile (42, 43) geschlossen ist, das andere Ventil (43, 42) offen ist, und umgekehrt.

17. Künstlicher Darmabschnitt (2) nach Anspruch 15 oder 16, wobei das zumindest eine Ventil ein Abteil mit einem variablen Volumen umfasst, das angepasst ist, das Ventil durch Verändern des Volumens des Abteils zu öffnen und zu schließen.

18. Künstlicher Darmabschnitt (2) nach einem der Ansprüche 1 bis 17, des Weiteren umfassend zumindest ein zusätzliches Ventil, das angepasst ist, einen Fluss von Darminhalten in einem natürlichen Abschnitt des Darms (70, 80) des Patienten stromaufwärts und/oder stromabwärts des künstlichen Darmabschnitts (2) zu steuern, wobei das zusätzliche Ventil angepasst ist, innerhalb des Körpers (100) des Patienten außerhalb eines Abschnitts des natürlichen Darms (70, 80) des Patienten implantiert zu werden, und zumindest ein Element umfasst, das angepasst ist, auf den natürlichen Darmabschnitt (70, 80) von außerhalb davon zu wirken, um einen Fluss von Darminhalten durch den natürlichen Darmabschnitt (70, 80) zu verhindern.

19. Künstlicher Darmabschnitt (2) nach einem der Ansprüche 14 bis 18, wobei die Flusssteuervorrichtung eine Pumpe (41) zum Fördern von Darminhalten durch den zweiten offenen Endteil (4) aus dem künstlichen Darmabschnitt (2) hinaus umfasst, und/oder wobei das zumindest eine Element zumindest einen Motor umfasst, der zum automatischen Antreiben zumindest eines Energie verbrauchenden Teils der Flusssteuervorrichtung eingerichtet ist.

20. System, umfassend den künstlichen Darmabschnitt (2) nach einem der Ansprüche 1 bis 19 und des Weiteren umfassend eine Energiequelle zum direkten oder indirekten Liefern von Energie zu zumindest einem Energie verbrauchenden Teil des Systems.

21. System nach Anspruch 20, wobei die Energiequelle einen drahtlosen Energieüberträger umfasst, der angepasst ist, Energie von außerhalb des Körpers (100) des Patienten zu dem zumindest einen Energie verbrauchenden Teil drahtlos zu übertragen, und des Weiteren umfassend ein Rückmeldungssubsystem, das angepasst ist, Rückmeldungsinformation bezüglich der in dem Akkumulator zu speichernden Energie von innerhalb des menschlichen Körpers nach außen drahtlos zu senden, wobei das System angepasst ist, die Rückmeldungsinformation zum Anpassen der Menge von von dem Energieüberträger übertragener drahtloser Energie zu verwenden.

22. System nach Anspruch 21, wobei die Rückmeldungsinformation mit einer Energiebilanz zusammenhängt, welche definiert ist als die Bilanz zwischen einer Menge von im menschlichen Körper empfangener drahtloser Energie und einer Menge von Energie, die von dem zumindest einen Energie verbrauchenden Teil verbraucht wird, oder wobei die Rückmeldungsinformation mit einer Energiebilanz zusammenhängt, welche definiert ist als die Bilanz zwischen einer Rate von im menschlichen Körper empfangener drahtloser Energie und einer Rate von Energie, die von dem zumindest einen Energie verbrauchenden Teil verbraucht wird.

23. System nach einem der Ansprüche 21 oder 22, umfassend eine implantierbare Energietransformationsvorrichtung zum Transformieren von drahtlos übertragener Energie in elektrische Energie.

24. System, umfassend ein künstliches Darmstück (2) nach einem der Ansprüche 1 bis 19 oder das System nach einem der Ansprüche 20 bis 23, des Weiteren umfassend eine Steuereinheit (90, 91), die angepasst ist, eines oder mehrere Elemente des Systems direkt oder indirekt zu steuern.

25. System nach Anspruch 24, umfassend einen manuell betätigbaren Schalter (48) zum Aktivieren der Steuereinheit (90, 91), wobei der Schalter (48) vorzugsweise zur subkutanen Implantation eingerichtet ist, um von außerhalb des Körpers (100) des Patienten betätigbar zu sein, wobei die Steuereinheit (90, 91) einen ersten Teil (91) umfasst, der zur Implantation in den Körper (100) des Patienten angepasst ist, und einen zweiten Teil (90), der angepasst ist, mit dem ersten Teil (91) von außerhalb des Körpers (100) des Patienten zusammenzuarbeiten, wobei der zweite Teil (90) der Steuereinheit (90, 91) angepasst ist, ein Steuersignal an den implantierbaren ersten Teil (91) der Steuereinheit (90, 91) zum Steuern des zumindest einen Energie verbrauchenden Teils von außerhalb des Körpers (100) des Patienten drahtlos zu übertragen.

26. System, umfassend ein künstliches Darmstück (2) nach einem der Ansprüche 1 bis 19 oder das System nach einem der Ansprüche 20 bis 25, des Weiteren umfassend einen Sensor für physikalische Parameter, der angepasst ist, einen physikalischen Parameter des Patienten direkt oder indirekt zu erfassen, wobei der Sensor für physikalische Parameter angepasst ist, zumindest einen der folgenden physikalischen Parameter des Patienten zu erfassen: einen Druck innerhalb des künstlichen Darmabschnitts (2), einen Druck innerhalb des Darms (70, 80) des Patienten, eine Ausdehnung des künstlichen Darmabschnitts (2), eine Dehnung einer Darmwand des Darms (70, 80) des Patienten, eine Bewegung der Darmwand des Patienten, und/oder des Weiteren umfassend einen Sensor für funktionelle Parameter, der angepasst ist, einen funktionellen Parameter des Systems direkt oder indirekt zu erfassen, wobei der Sensor für funktionelle Parameter angepasst ist, zumindest einen der folgenden funktionellen Parameter des Systems zu erfassen: einen Druck gegen einen Teil des Systems, wie den künstlichen Darmabschnitt (2), eine Dehnung eines Teils des Systems, wie einer Wand des künstlichen Darmabschnitts (2), einen elektrischen Parameter, wie Spannung, Stromstärke oder Energiebilanz, eine Position oder Bewegung eines beweglichen Teils des Systems.

## Revendications

1. Section d'intestin artificiel (2) apte à être implantée à l'intérieur d'un corps (100) de patient, ladite section d'intestin (2) présentant une première portion à extrémité ouverte (3) et une seconde portion à extrémité ouverte (4) permettant une communication d'écoulement entre l'une et l'autre, et comprenant en outre au moins un élément apte à interagir directement ou indirectement avec le contenu intestinal contenu dans la section d'intestin artificiel (2) entre les première et seconde portions à extrémité ouverte de celle-ci (3, 4), dans laquelle le au moins un élément comprend un réservoir artificiel (40) entre les première et seconde portions à extrémité ouverte (3, 4) pour recevoir et collecter temporairement dans celui-ci un contenu intestinal fourni à travers la première portion à extrémité ouverte (3), **caractérisée en ce qu'**au moins la première portion à extrémité ouverte (3) est apte à être reliée à une ouverture latérale créée par voie chirurgicale dans une paroi de l'intestin (70, 80) du patient.

2. Section d'intestin artificiel (2) selon la revendication 1, dans laquelle la seconde portion à extrémité ouverte (4) est apte à être reliée à une stomie créée par voie chirurgicale (170), ou au rectum ou à l'anus du patient, ou à un tissu adjacent à l'anus du patient.

3. Section d'intestin artificiel (2) selon la revendication 1, dans laquelle la seconde portion à extrémité ouverte (4) est apte à être reliée à l'intestin grêle (70) d'un patient ou au gros intestin (80) d'un patient.

4. Section d'intestin artificiel (2) selon l'une quelconque des revendications 1 à 3, dans laquelle la première et la seconde portions à extrémité ouverte (3,4) sont toutes deux aptes à être reliées à une ouverture latérale créée par voie chirurgicale dans une paroi de l'intestin (70, 80) du patient, ou dans laquelle la seconde portion à extrémité ouverte (4) est apte à être reliée à une ouverture en travers de l'intestin (70, 80) du patient.

5. Section d'intestin artificiel (2) selon l'une quelconque des revendications 1 à 4, dans laquelle au moins la première portion à extrémité ouverte (3) comprend une portion d'épaulement (23) formée autour de la portion d'extrémité en vue d'une liaison latérale sur la paroi intestinale du patient, dans laquelle au moins une partie de la portion d'épaulement (23) s'étend latéralement à partir de la section d'intestin artificiel (2) à raison de 3 mm à 20 mm, dans laquelle la portion d'épaulement (23) présente une section incurvée, de manière à se conformer de manière générale à une paroi intestinale lorsqu'elle est attachée latéralement à celle-ci, dans laquelle la portion d'épaulement (23) est divisée en une portion d'épaulement supérieure (24) et inférieure (25) avec un espace (26) entre les portions d'épaulement supérieure (24) et inférieure (25) apte à loger à l'intérieur un tissu de paroi intestinale, dans laquelle la portion d'épaulement inférieure (25) est apte à être placée à l'intérieur de l'intestin (70, 80) du patient à travers une ouverture latérale créée par voie chirurgicale (72) dans la paroi intestinale, et dans laquelle la portion d'épaulement supérieure (24) est apte à être placée à l'extérieur de la paroi intestinale.

6. Section d'intestin artificiel (2) selon l'une quelconque des revendications 1 à 5, dans laquelle au moins la première portion à extrémité ouverte (3) est apte à être reliée à la paroi intestinale du patient par collage, suture ou agrafage.

7. Section d'intestin artificiel (2) selon l'une quelconque des revendications 1 à 6, dans laquelle au moins la première portion à extrémité ouverte (3) est fabriquée dans un matériau biocompatible, dans laquelle le matériau biocompatible de la portion à extrémité ouverte (3) comprend au moins un matériau parmi le groupe suivant de matériaux : titane, acier inoxydable, céramique, matériau polymère biocompatible, le matériau polymère biocompatible comprenant au moins un polymère du groupe suivant de polymères : polytétrafluoroéthylène, silicone, polyruéthane, polytétrafluoroéthylène expansé (ePTFE).

8. Section d'intestin artificiel (2) selon l'une quelconque des revendications 1 à 7, dans laquelle au moins la première portion à extrémité ouverte (3) comprend un matériau multicouche.

9. Section d'intestin artificiel (2) selon l'une quelconque des revendications 1 à 8, dans laquelle au moins la première portion à extrémité ouverte (3) comprend une couche de repousse poreuse permettant la repousse de tissu vivant.

10. Section d'intestin artificiel (2) selon l'une quelconque des revendications 1 à 9, comprenant un manchon flexible (10) apte à s'étendre sur à la fois la seconde portion à extrémité ouverte (4) et une ouverture en travers de l'intestin (70, 80) du patient, de telle sorte qu'une partie de l'intestin (70, 80) se situe de manière intermédiaire par rapport au manchon (10) et une surface extérieure (6) de la seconde portion à extrémité ouverte (4), dans laquelle le manchon (10) sert à améliorer la robustesse de la liaison face aux forces axiales résultant de mouvements péristaltiques d'un intestin (70, 80), lesquels ont tendance à tirer sur l'intestin (70, 80), à l'état implanté.

11. Section d'intestin artificiel (2) selon la revendication 10, dans laquelle ledit manchon flexible (10) est installé sur une surface extérieure (6) de la seconde portion à extrémité ouverte (4) de manière à pouvoir être replié sur lui-même, de telle sorte que l'intestin (70, 80) du patient puisse se situer de manière intermédiaire dans le manchon replié (10).

12. Section d'intestin artificiel (2) selon la revendication 10 ou 11, dans laquelle le manchon (10) est roulé sur lui-même et peut être déroulé, de telle sorte que la partie (81) de l'intestin (80) se situe de manière intermédiaire par rapport au manchon (10) et la surface extérieure (6) de la seconde portion à extrémité ouverte (4).

13. Section d'intestin artificiel (2) selon l'une quelconque des revendications 1 à 12, comprenant :
- un renflement (15) formé sur la surface extérieure de la seconde portion à extrémité ouverte (4) pour une partie de l'intestin (70, 80) du patient présentant une ouverture en travers devant être étendue sur le renflement (15) à partir d'un côté du renflement (15), et
- un anneau de blocage (30) apte à être avancé sur la partie d'intestin à partir de l'autre côté respectif du renflement (15), de telle sorte que la partie d'intestin se situe de manière intermédiaire par rapport à la surface extérieure du morceau d'intestin artificiel et de l'anneau de blocage (30).

14. Section d'intestin artificiel (2) selon l'une quelconque des revendications 1 à 13, dans laquelle le au moins un élément comprend un dispositif de commande d'écoulement apte à commander l'écoulement du contenu intestinal à partir de la section d'intestin artificiel (2) à travers la seconde portion à extrémité ouverte (4), dans laquelle le dispositif de commande d'écoulement est apte à empêcher l'écoulement du contenu intestinal à partir de la section d'intestin artificiel (2) à travers la seconde portion à extrémité ouverte (4).

15. Section d'intestin artificiel (2) selon la revendication 14, dans laquelle le dispositif de commande d'écoulement comprend au moins une valve, incluant une valve de sortie (43) empêchant l'écoulement du contenu intestinal à travers la seconde portion à extrémité ouverte (4) dans sa position fermée.

16. Section d'intestin artificiel (2) selon la revendication 14 ou 15, dans laquelle le dispositif de commande d'écoulement comprend une valve d'entrée (42) permettant l'écoulement du contenu intestinal à travers la première portion à extrémité ouverte (3) vers la section d'intestin artificiel (2) dans sa position ouverte, dans laquelle la section d'intestin artificiel (2) comprend en outre une unité de commande (90, 91) destinée à commander la valve de sortie (43) et la valve d'entrée (42), de telle sorte que lorsque l'une des deux valves (42, 43) est fermée, l'autre valve (43, 42) est ouverte, et inversement.

17. Section d'intestin artificiel (2) selon la revendication 15 ou 16, dans laquelle la au moins une valve comprend un compartiment avec un volume variable apte à ouvrir et fermer la valve en modifiant le volume du compartiment.

18. Section d'intestin artificiel (2) selon l'une quelconque des revendications 1 à 17, comprenant en outre au moins une valve supplémentaire apte à commander l'écoulement de contenu intestinal dans une section naturelle de l'intestin (70, 80) d'un patient en amont et/ou aval de la section d'intestin artificiel (2), dans laquelle la valve supplémentaire est apte à être implantée à l'intérieur du corps (100) du patient à l'extérieur d'une section de l'intestin naturel (70, 80) du patient, et comprend au moins un élément apte à agir sur la section d'intestin naturel (70, 80) à partir de l'extérieur de celle-ci de manière à empêcher l'écoulement de contenu intestinal à travers la section d'intestin naturel (70, 80).

19. Section d'intestin artificiel (2) selon l'une quelconque des revendications 14 à 18, dans laquelle le dispositif de commande d'écoulement comprend une pompe (41) destinée à faire avancer un contenu intestinal à travers la seconde portion à extrémité ouverte (4) vers l'extérieur de la section d'intestin artificiel (2), et/ou dans laquelle le au moins un élément comprend au moins un moteur agencé pour entraîner automatiquement au moins une partie consommant de l'énergie du dispositif de commande d'écoulement.

20. Système comprenant la section d'intestin artificiel (2) selon l'une quelconque des revendications 1 à 19, et comprenant en outre une source d'énergie destinée à fournir directement ou indirectement de l'énergie à au moins une partie consommant de l'énergie du système.

21. Système selon la revendication 20, dans lequel la source d'énergie comprend un transmetteur d'énergie sans fil apte à transmettre de manière sans fil de l'énergie à partir de l'extérieur du corps (100) du patient à la au moins une partie consommant de l'énergie, et comprenant en outre sous-système de réaction destiné à envoyer de manière sans fil des informations de réaction liées à l'énergie devant être stockée dans l'accumulateur de l'intérieur du corps humain vers l'extérieur de celui-ci, dans lequel le système est apte à utiliser les informations de réaction pour adapter la quantité d'énergie sans fil transmise par le transmetteur d'énergie.

22. Système selon la revendication 21, dans lequel les informations de réaction sont liées à un bilan énergétique défini comme le bilan entre une quantité d'énergie sans fil reçue à l'intérieur du corps humain et une quantité d'énergie consommée par la au moins une partie consommant de l'énergie, et/ou dans lequel les informations de réaction sont liées à un bilan énergétique défini comme le bilan entre un taux d'énergie sans fil reçue à l'intérieur du corps humain et un taux d'énergie consommée par la au moins une partie consommant de l'énergie.

23. Système la revendication 21 ou 22, comprenant un dispositif transformant l'énergie implantable destiné à transformer de manière sans fil une énergie transmise en énergie électrique.

24. Système comprenant un morceau d'intestin artificiel (2) selon l'une quelconque des revendications 1 à 19, ou le système selon l'une quelconque des revendications 20 à 23, comprenant en outre une unité de commande (90, 91) apte à commander directement ou indirectement un ou plusieurs éléments du système.

25. Système selon la revendication 24, comprenant un commutateur pouvant être actionné manuellement (48) destiné à activer l'unité de commande (90, 91), le commutateur (48) étant agencé de préférence pour une implantation sous-cutanée de manière à pouvoir être actionné à partir de l'extérieur du corps (100) du patient, dans lequel l'unité de commande (90, 91) comprend une première partie (91) apte à une implantation dans le corps (100) du patient et une seconde partie (90) apte à coopérer avec la première partie (91) à partir de l'extérieur du corps (100) du patient, dans lequel la seconde partie (90) de l'unité de commande (90, 91) est apte à transmettre de manière sans fil un signal de commande à la première partie implantable (91) de l'unité de commande (90, 91) pour commander la au moins une partie consommant de l'énergie à partir de l'extérieur du corps (100) du patient.

26. Système comprenant un morceau d'intestin artificiel (2) selon l'une quelconque des revendications 1 à 19, ou le système selon l'une quelconque des revendications 20 à 25, comprenant en outre un capteur de paramètre physique apte à détecter directement ou indirectement un paramètre physique du patient, dans lequel le capteur de paramètre physique est apte à détecter au moins un des paramètres physiques suivants du patient : une pression à l'intérieur de la section d'intestin artificiel (2), une pression à l'intérieur de l'intestin (70, 80) du patient, une dilatation de la section d'intestin artificiel (2), une distension de la paroi intestinale de l'intestin (70, 80) du patient, un mouvement de la paroi intestinale, et/ou comprenant un capteur de paramètre fonctionnel apte à détecter directement ou indirectement un paramètre fonctionnel du système, dans lequel le capteur de paramètre fonctionnel est apte à détecter au moins un des paramètres fonctionnels suivants du système : une pression contre une partie du système comme la section d'intestin artificiel (2), une distension d'une partie du système comme une paroi de la section d'intestin artificiel (2), un paramètre électrique comme la tension, le courant ou le bilan énergétique, une position ou un mouvement d'une partie pouvant être déplacée du système.
